(19) Europäisches Patentamt
European Patent Office
Office européen des brevets

(11) EP 3 426 355 B1

(12) FASCICULE DE BREVET EUROPEEN

(45) Date de publication et mention de la délivrance du brevet:
**27.01.2021 Bulletin 2021/04**

(21) Numéro de dépôt: **17710511.1**

(22) Date de dépôt: **07.03.2017**

(51) Int Cl.:
***A61Q 19/08*** $^{(2006.01)}$ ***A61K 8/92*** $^{(2006.01)}$
***A61K 8/97*** $^{(2017.01)}$

(86) Numéro de dépôt international:
**PCT/EP2017/055350**

(87) Numéro de publication internationale:
**WO 2017/153422 (14.09.2017 Gazette 2017/37)**

# (54) CORPS GRAS ACTIVÉ ET/OU CIRE ACTIVÉE AU MOYEN DE LA FRACTION NON HYDROSOLUBLE DU SUC DE CARICA PAPAYA

AKTIVIERTER FETTSTOFF UND/ODER AKTIVIERTER WACHS, DER DURCH DIE WASSERUNLÖSLICHE FRAKTION DES PFLANZENSAFTS CARICA PAPAYA GEWONNEN WIRD

ACTIVATED FATTY SUBSTANCE AND/OR ACTIVATED WAX OBTAINED THROUGH THE ACTION OF THE WATER-INSOLUBLE FRACTION OF CARICA PAPAYA JUICE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **08.03.2016 FR 1600394**

(43) Date de publication de la demande:
**16.01.2019 Bulletin 2019/03**

(73) Titulaire: **ISP Investments LLC.**
**Wilmington, DE 19805 (US)**

(72) Inventeur: **COQUET, Corinne**
**06620 Cipieres (FR)**

(74) Mandataire: **Miquel, Corinne**
**Ashland Specialties France**
**655, route du Pin Montard**
**BP 212**
**06904 Sophia Antipolis Cedex (FR)**

(56) Documents cités:
**FR-A1- 2 853 834**

- **RIVERA I1 ET AL: "Plant lipases: partial purification of Carica papaya lipase", METHODS MOL BIOL,, vol. 861, 1 janvier 2012 (2012-01-01), pages 115-122, XP009191177,**
- **ROGER GIORDANI, ANDRE MOULIN, ROBERT VERGER: "Tributyroylglycerol hydrolase activity in Carica papaya and other latices", PHYTOCHEMISTRY, vol. 30, no. 4, 1991, - 1991, pages 1069-1072, XP002761988, DOI: 10.1016/S0031-9422(00)95174-4**
- **VILLENEUVE P ET AL: "Carica papaya latex lipase:sn-3 stereoselectivity or short-chain selectivity? Model chiral triglycerides are removing the ambiguity", JOURNAL OF THE AMERICAN OIL CHEMISTS' SOCIETY (JAOCS), vol. 72, no. 6, 1 juin 1995 (1995-06-01), pages 753-755, XP009191178, SPRINGER, DE ISSN: 0003-021X**
- **Kumar D. Mukherjee: "Lipase-catalyzed synthesis of designer lipids with improved nutritional properties", Food, Nutrition and Well Being , 18 octobre 1998 (1998-10-18), 20 octobre 1998 (1998-10-20), pages 167-169, XP002761989, 3RD KARLSRUHE NUTRITION SYMPOSIUM EUROPEAN RESEARCH TOWARDS SAFER AND BETTER FOOD REVIEW AND TRANSFER CONGRESS, KARLSRUHE, GERMANY Extrait de l'Internet: URL:https://publikationen.bibliothek.kit.e du/1000011778/867306 [extrait le 2016-09-19]**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

(Cont. page suivante)

- Fernanda Wiermann PAQUES et al.: "Characterization of the lipase from Carica papaya residues", Braz. J. Food Technol., vol. 11, no. 1 2008, mars 2008 (2008-03), pages 20-27, XP002761990, Extrait de l'Internet: URL:http://www.ital.sp.gov.br/bj/artigos/bjft/2008/v11n12407-21.pdf [extrait le 2016-07]
- PABLO DOMÍNGUEZ DE MARÍA, JOSÉ V. SINISTERRA, SHAU-WEI TSAI, ANDRÉS R. ALCÁNTARA: "Carica papaya lipase (CPL): An emerging and versatile biocatalyst", BIOTECHNOLOGY ADVANCES, vol. 24, no. 5, septembre 2006 (2006-09), - octobre 2006 (2006-10), pages 493-499, XP002761991, DOI: 10.1016/j.biotechadv.2006.04.002

## Description

### Domaine technique

La présente invention concerne le domaine du cosmétique. Plus particulièrement, l'invention a trait à des corps gras activés et/ou cires activées administrables par application/voie cutanée, lesdits corps gras activés et/ou cires activées étant utiles en cosmétique, notamment pour retarder l'apparition ou limiter les signes vieillissement cutané, protéger la peau et/ou les phanères contre tous types d'agressions extérieures, et/ou favoriser la différenciation cellulaire au niveau cutané, ou encore renforcer la fonction barrière de la peau et/ou des phanères.

### Etat de la technique

La peau est un organe vital assurant des fonctions multiples telles que des fonctions sensitives, protectrices vis à vis d'agressions externes multiples (fonction barrière), immunitaires, métaboliques ou encore thermorégulatrices. Ces rôles sont rendus possibles grâce à une structure en trois compartiments distincts superposés : l'épiderme, le derme et l'hypoderme. L'épiderme est un épithélium de revêtement qui constitue la structure externe de la peau et assure sa fonction de protection. Cette fonction est assurée par la cohésion des cellules épithéliales et par la production d'une protéine filamenteuse et résistante, la kératine. Le derme est un tissu conjonctif constitué d'une substance fondamentale dans laquelle baignent les fibroblastes, des fibres de collagène et des fibres d'élastine, fibres protéiques synthétisées par les fibroblastes. Les fibres de collagène assurent une grande partie de la solidité du derme, elles participent à l'élasticité et surtout à la tonicité de la peau et/ou des muqueuses. Au-dessous du derme se trouve une couche de tissus adipeux : l'hypoderme.

La peau, comme tous les autres organes, est soumise au vieillissement. Les facteurs qui interviennent dans le processus du vieillissement cutané sont variés et nombreux. Le phénomène de vieillissement chronologique de la peau s'accompagne, entre autres, d'un ralentissement des fonctions cellulaires et l'apparition d'anomalies structurales dans certaines protéines de la matrice extracellulaire. La peau devient moins souple, plus fine, souvent sèche et elle perd son élasticité, ce qui s'accompagne de rides et de ridules.

Dans l'épiderme, le ralentissement de la prolifération et de la différenciation des kératinocytes avec l'âge est un facteur permettant d'expliquer l'amincissement de la peau au cours du vieillissement. L'épiderme s'atrophie, la peau perd ainsi ses capacités de protection. Les fibroblastes dermiques sont également altérés, et on observe une diminution de la production des protéines de la matrice extracellulaire, telles que le collagène ou l'élastine.

Une exposition chronique aux ultra-violets et à d'autres facteurs environnementaux agressifs, accélère et aggrave le phénomène de vieillissement de la peau. On parle de photo-vieillissement.

Un des mécanismes jouant un rôle majeur dans le processus du vieillissement est l'accumulation de dommages oxydatifs dans des molécules essentielles telles que dans les membranes lipidiques, les protéines, l'ADN, plus particulièrement l'ADN mitochondrial. Ainsi, une des conséquences importantes de l'accumulation de ces dommages est une réduction de la capacité de la cellule à produire de l'ATP.

Les professionnels de la santé et du cosmétique recherchent depuis de nombreuses années des moyens pour lutter ou, tout au moins, pour réduire les phénomènes du vieillissement cutané, ainsi que des moyens afin d'augmenter la résistance de la peau aux agressions extérieures et au stress qu'elle subit quotidiennement.

En cosmétique, les huiles sont à la base du concept de cosmétique naturelle. Les huiles végétales, obtenues par traitement naturel sans adjonction de produits chimiques de synthèse sont utilisées traditionnellement pour leur propriété d'hydratation et de protection.

De par leur composition spécifique, les huiles les plus utilisées en cosmétique sont des huiles composées d'acides polyinsaturés à longue chaîne, notamment gamma-linolénique qui joue un rôle spécifique sur la fluidité des membranes, la réduction des pertes hydriques trans-épidermiques, la prévention du vieillissement de la peau. En effet, certains acides gras sont susceptibles d'améliorer ou de conserver le degré d'hydratation de la peau.

Les huiles sont le plus souvent utilisées en tant qu'excipient et non en tant que principe actif. Toutefois, la Demanderesse a précédemment développé une composition, notamment utile à des fins cosmétiques, comprenant, comme principe actif, dans un milieu physiologiquement acceptable, au moins l'association d'un diacylglycérol (abrégé en « DAG ») spécifique et d'un alcool gras particulier. Cette composition est décrite et revendiquée dans la demande de brevet PCT/FR2004/000944, publiée sous la référence WO 2004/093832 A2, au nom de la Demanderesse.

Les diacylglycérols (DAG) sont des composés notamment décrits dans l'état de la technique pour leurs propriétés de marqueur. Ils jouent un rôle fondamental, notamment par l'action qu'ils exercent sur une protéine kinase C, et ont ainsi une fonction d'activateur cellulaire.

La méthode réactionnelle, divulguée dans la demande de brevet WO 2004/093832, afin d'obtenir les DAG, peut être une réaction chimique effectuée en milieu acide ou alcalin comme, par exemple, une hydrolyse des matières grasses constituées de triglycérides. Cette hydrolyse peut encore être réalisée de manière enzymatique comme, par

exemple, une réaction biochimique qui utilise une hydrolase telle qu'une lipase. Préférentiellement, les DAG utilisés dans les compositions de WO 2004/093832 sont obtenus à partir de matières grasses (huiles animales, végétales ou de synthèse) comprenant des triglycérides ayant subi une hydrolyse enzymatique, notamment par des lipases.

Les lipases sont des enzymes qui hydrolysent les esters du glycérol. On détermine plusieurs types de lipase en fonction de leur différent niveau de vitesse de réaction. On peut aussi classer ces enzymes en plusieurs groupes, en fonction de leurs différentes spécificités : spécificité vis à vis du substrat ; spécificité de position ou régiosélective ; spécificité vis à vis de la nature des acides gras ou typosélectivité ; spécificité vis à vis d'une position ou stéréospécificité.

Certaines lipases n'ont pas de spécificité particulière. D'autres sont régiosélectives : ainsi, par exemple, la lipase pancréatique et les lipases d'origine microbienne, d'Aspergillus, de Rhysopus ou encore de Rhizomucor miehei, sont des lipases 1,3 selectives. Les lipases régiosélectives sont d'un usage industriel courant aussi bien dans l'industrie des corps gras que dans les détergents. D'autres lipases sont stéréospécifiques comme la lipase de Candida qui possède une stéréospécificité de type sn2. La nomenclature sn détermine les positions sn1, sn2 et sn3 du squelette des triglycérides en fonction de la configuration du carbone asymétrique présenté en projection de Fisher.

Dans WO 2004/093832, une lipase stéréospécifique de type sn3 (à savoir qui permet l'hydrolyse des triglycérides en position 3) est avantageusement utilisée. Cette lipase hydrolyse ainsi les triglycérides en position 3 en formant préférentiellement du 1,2-DAG. La lipase utilisée peut être d'origine végétale (Carica papaya) ou microbienne (Penicellium cyclopium). Selon un mode de réalisation préféré, l'hydrolyse des triglycérides est réalisée dans WO 2004/093832 avec une lipase de Carica papaya (Villeneuve et al. JAOCS, 72, 6:753.1995).

Toutefois, bien que les compositions obtenues en mettant en œuvre l'enseignement de la demande de brevet WO 2004/093832 possèdent notamment des propriétés cosmétiques satisfaisantes au niveau cutané, la Demanderesse s'efforce, depuis plus de dix années déjà, à améliorer le procédé de préparation de ces compositions, lequel procédé est exemplifié au sein de l'exemple 1 de WO 2004/093832. Plus précisément, et tel qu'indiqué au sein de cet exemple 1, la réaction d'hydrolyse enzymatique permettant d'obtenir les 1,2-DAG est possible avec du suc de papaye brut, toutefois il est recommandé d'utiliser une préparation purifiée de ce suc après solubilisation en milieu aqueux, conservée dans un polyol ou lyophilisée. En effet, le procédé de préparation objet de l'exemple 1 de WO 2004/093832, s'il fonctionne, présente un certains nombres de difficultés pratiques. En effet, concernant l'utilisation d'un suc de papaye brut, ceci est notamment déconseillé au regard du fait que le suc de papaye brut contient non seulement la lipase d'intérêt mais également des protéases (parmi lesquelles figure la papaïne, une protéase à cystéine) susceptibles de dégrader la lipase ou, à tout le moins, d'impacter négativement son activité enzymatique.

C'est donc en toute logique que l'exemple 1 de WO 2004/093832 privilégie l'utilisation d'une préparation purifiée de ce suc après solubilisation en milieu aqueux, conservée dans un polyol (par exemple dans du sorbitol) ou lyophilisée. Concernant cette dernière possibilité, il est connu de l'homme du métier que les lyophilisats présentent un certain nombre d'inconvénients, parmi lesquels figurent leur sensibilité extrême à l'humidité atmosphérique et leur procédé de fabrication complexe (et, par conséquent, onéreux).

Concernant la solution technique consistant à utiliser une préparation purifiée du suc de papaye après solubilisation en milieu aqueux et conservation dans un polyol (tel que le sorbitol), il s'avère que cette technique présente notamment l'inconvénient de nécessiter un traitement complexe postérieurement à l'hydrolyse partielle des triglycérides. En effet, afin de stopper la réaction d'hydrolyse partielle des triglycérides, il s'avère nécessaire d'effectuer plusieurs étapes de lavage du milieu aqueux contenant la lipase de Carica papaya conservée dans le polyol, notamment en utilisant des solvants organiques.

En outre, la Demanderesse a découvert que la lipase de Carica papaya solubilisée en milieu aqueux et conservée dans un polyol souffrait de la congélation et, par conséquent, pouvait difficilement - voire ne pouvait pas - être conservée sous forme congelée, alors que ceci s'avérerait éminemment souhaitable, en vue de garantir une conservation dans le temps de bonne qualité, pratique et peu onéreuse de la lipase de Carica papaya.

## Exposé de l'invention

A force de recherches, la Demanderesse a mis au point une véritable plateforme technologique permettant de remédier à tout ou partie des inconvénients susmentionnés. Cette plateforme technologique est principalement basée sur l'obtention et l'utilisation de la fraction non hydrosoluble (insoluble dans l'eau) du suc de Carica papaya. Par conséquent, la présente invention a pour objet un procédé d'obtention de la fraction non hydrosoluble du suc de Carica papaya, enrichie en lipase de Carica papaya, ledit procédé comprenant les étapes suivantes :

a) mettre en suspension du suc de *Carica papaya* brut séché dans de l'eau distillée, avantageusement dans un rapport en poids *Carica papaya* brut séché/eau distillée compris entre environ 0,01 et environ 0,5 (de préférence entre 0,01 et 0,5), de préférence entre environ 0,05 et environ 0,25 (de préférence entre 0,05 et 0,25), préférablement entre environ 0,08 et environ 0,2 (de préférence entre 0,08 et 0,2); et de manière particulièrement préférée d'environ 0,1 (de préférence de 0,1),

b) centrifuger la suspension obtenue à l'étape a) de manière à obtenir un culot contenant ladite fraction non hydrosoluble du suc de *Carica papaya,* avantageusement durant un intervalle de temps compris entre environ 5 minutes et environ 90 minutes (de préférence entre 5 minutes et 90 minutes), préférablement compris entre environ 15 minutes et environ 60 minutes (de préférence entre 15 minutes et 60 minutes), de manière préférée compris entre environ 20 et environ 40 minutes (de préférence entre 20 et 40 minutes), et avantageusement à une vitesse de rotation comprise entre environ 2000 et environ 6000 rpm (de préférence entre 2000 et 6000 rpm), préférablement comprise entre environ 3000 et environ 5000 rpm (de préférence entre 3000 et 5000 rpm), de manière préférée d'environ 4000 rpm (de préférence de 4000 rpm).

c) récupérer le culot contenant ladite fraction non hydrosoluble du suc de *Carica papaya,* enrichie en lipase de *Carica papaya.*

De manière particulièrement avantageuse, ledit procédé comprend, après l'étape a) et avant l'étape b), l'étape suivante :

a') agiter la suspension obtenue à l'étape a) durant un intervalle de temps compris entre environ 15 minutes et environ 240 minutes (de préférence entre 15 minutes et 240 minutes), avantageusement durant un intervalle de temps compris entre environ 30 minutes et environ 180 minutes (de préférence entre 30 minutes et 180 minutes), préférablement entre environ 60 minutes et environ 150 minutes (de préférence entre 60 minutes et 150 minutes), et de manière particulièrement préférée d'environ 120 minutes (de préférence de 120 minutes), et à une température comprise entre environ 10°C et environ 30°C (de préférence entre 10°C et 30°C), avantageusement à une température comprise entre environ 15°C et environ 25°C (de préférence entre 15°C et 25°C), de préférence à température ambiante.

Par température ambiante, l'on entend une température comprise entre environ 20°C et environ 25°C (de préférence entre 20°C et 25°C), avantageusement une température d'environ 20°C (de préférence de 20°C).

De préférence, ledit procédé comprend, après l'étape b) et avant l'étape c), l'étape suivante :
b') sécher le culot humide jusqu'à obtention d'une poudre particulaire ; la fraction non hydrosoluble du suc de *Carica papaya,* enrichie en lipase de *Carica papaya,* étant récupérée, à l'étape c), sous forme de poudre particulaire.

Un autre objet de l'invention concerne la fraction non hydrosoluble du suc de Carica papaya enrichie en lipase de Carica papaya, susceptible d'être obtenue par le procédé susvisé.

L'invention concerne également la fraction non hydrosoluble du suc de Carica papaya enrichie en lipase de Carica papaya, directement obtenue par le procédé susvisé.

De manière particulièrement avantageuse, la Demanderesse a découvert que cette fraction non hydrosoluble du suc de Carica papaya enrichie en lipase de Carica papaya, pouvait être simplement conservée par congélation. En outre, le procédé d'obtention de cette fraction non hydrosoluble du suc de Carica papaya enrichie en lipase de Carica papaya, est extrêmement simple à mettre en œuvre et peu onéreux.

L'invention concerne également l'utilisation de la fraction non hydrosoluble du suc de Carica papaya selon la présente invention pour enrichir un corps gras en diacylglycérols (et obtenir ainsi un corps gras activé) et/ou une cire en alcools gras (et obtenir ainsi une cire activée).

Un autre objet de l'invention concerne un procédé de préparation d'un corps gras enrichi en diacylglycérols (corps gras activé), ledit procédé comprenant les étapes suivantes :

i) mélanger un corps gras avec de l'eau ou avec une solution saline contenant de préférence un ion bivalent, tel qu'un ion calcium ou magnésium, dans un rapport en volume eau ou solution saline/corps gras compris entre environ 0,005 et environ 0,5 (de préférence entre 0,005 et 0,5), préférablement entre environ 0,01 et environ 0,4 (de préférence entre 0,01 et 0,4),

ii) maintenir ce mélange sous agitation (de préférence sous agitation vive, préférablement à une vitesse d'environ 2000 à environ 3000 rpm (de préférence de 2000 à 3000 rpm); ladite agitation vive étant avantageusement obtenue au moyen d'un appareil favorisation les émulsions) jusqu'à obtention d'une émulsion entre le corps gras et l'eau, à une température comprise entre environ 30°C et environ 70°C (de préférence entre 30°C et 70°C), avantageusement d'environ 50°C (de préférence de 50°C), de préférence durant un intervalle de temps compris entre environ 1 et environ 30 minutes (de préférence entre 1 et 30 minutes), préférablement entre environ 5 et environ 20 minutes (de préférence entre 5 et 20 minutes), et de préférence d'environ 10 minutes (de préférence de 10 minutes),

iii) toujours sous agitation (de préférence sous agitation modérée, préférablement à une vitesse d'environ 200 à environ 300 rpm [de préférence de 200 à 300 rpm]) et à ladite température, mettre en présence le mélange obtenu à l'étape précédente avec un volume donné de la fraction non hydrosoluble du suc de *Carica papaya,* enrichie en lipase de *Carica papaya,* obtenue en mettant en œuvre le procédé d'obtention de la fraction non hydrosoluble du suc de *Carica papaya* susmentionné, dans un rapport en volume fraction non hydrosoluble du suc de *Carica papaya*/corps gras utilisé compris entre environ 0,01 et environ 0,2 (de préférence entre 0,01 et 0,2), avantageusement entre environ 0,05 et 0,015 (de préférence entre 0,05 et 0,015), de préférence d'environ 0,1 (de préférence de 0,1),

iv) maintenir ladite température et l'agitation durant un intervalle de temps compris entre environ une heure et environ six heures (de préférence entre une heure et six heures), avantageusement entre environ deux heures et environ cinq heures (de préférence entre deux heures et cinq heures), de préférence durant environ quatre heures (de préférence durant quatre heures), afin d'obtenir le corps gras enrichi en diacylglycérols.

De manière particulièrement préféré, ce procédé de préparation d'un corps gras enrichi en diacylglycérols (corps gras activé) comprend, après l'étape iv), l'étape suivante :
v) filtrer le mélange obtenu à l'étape iv), de préférence en utilisant au moins un filtre à gradient ou au moins une superposition d'au moins deux filtres de porosité décroissante, avantageusement d'environ 500 μm à environ 250 μm (de préférence de 500 μm à 250 μm), de façon à séparer ladite fraction non hydrosoluble du suc de *Carica papaya,* enrichie en lipase de *Carica papaya,* du milieu réactionnel contenant le corps gras enrichi en diacylglycérols.
De préférence, ledit procédé comprend, après l'étape v), au moins l'une des deux étapes suivantes, et avantageusement les deux :

- éliminer l'eau résiduelle présente dans le milieu réactionnel contenant le corps gras enrichi en diacylglycérols au moyen d'un agent dessiccateur tel que du sulfate de magnésium anhydre, et/ou
- désodoriser et/ou améliorer la brillance du corps gras enrichi en diacylglycérols au moyen d'un charbon actif.

Cette étape v) de filtration permet de stopper aisément la réaction d'hydrolyse partielle des triglycérides contenus dans le corps gras sans nécessiter une pluralité d'étapes de lavage de milieu aqueux contenant la lipase conservée dans un polyol (comme cela est le cas concernant le procédé de l'exemple 1 de WO 2004/093832) et permet d'éviter le recours à des solvants organiques. Suite à cette étape de filtration v), la fraction non hydrosoluble du suc de Carica papaya est également récupérée, de manière aisée, et peut être réutilisée.
D'une manière générale, le fait de pouvoir utiliser la fraction non hydrosoluble du suc de Carica papaya obtenue à l'aide du procédé d'obtention susvisé, rend la manipulation post hydrolyse partielle du corps gras relativement aisée. En outre, la Demanderesse a découvert que l'utilisation de cette fraction non hydrosoluble du suc de Carica papaya, enrichie en lipase de Carica papaya permettrait de préserver les acides gras insaturés, lesquels possèdent des propriétés cosmétiques avantageuses.
Selon un mode de réalisation particulier, la fraction non hydrosoluble du suc de Carica papaya, enrichie en lipase de Carica papaya filtrée à l'étape v), est réutilisée à l'étape iii) du procédé de préparation d'un corps gras enrichi en diacylglycérols selon l'invention.
De préférence, ledit corps gras est une huile d'origine animale, végétale et/ou marine, vierge ou raffinée, avantageusement vierge, ladite huile étant préférablement d'origine végétale et/ou marine, de préférence d'origine végétale.
Il est particulièrement important de noter que le fait d'utiliser la fraction non hydrosoluble du suc de Carica papaya permet de « travailler » avec une huile vierge, à savoir d'enrichir (activer) une huile vierge en diacylglycérols, ce qui est notamment souhaitable pour des raisons de sécurité sanitaire et d'innocuité (absence de solvants organiques liés au raffinement des huiles etc.). En outre, le procédé de préparation d'un corps gras enrichi en diacylglycérols selon l'invention est particulièrement adapté à l'enrichissement des huiles vierges, dans la mesure où la fraction insaponifiable de l'huile vierge n'est pas détériorée par ledit procédé, l'activité enzymatique utilisée étant de type triacylglycérol estérase. Ceci permet préserver l'activité biologique des huiles vierges à l'issue du procédé d'enrichissement en diacylglycérols ; cette activité biologique étant de surcroît améliorée par ledit procédé.
Préférablement, ledit corps gras est une huile comprenant des triglycérides possédant :

des chaînes hydrocarbonées aliphatiques d'un acide gras possédant un nombre de carbone compris entre 12 et 26, avantageusement entre 16 et 20, et de manière particulièrement préférée entre 16 et 18 ; ladite chaîne hydrocarbonée aliphatique étant linéaire ou ramifiée, avantageusement linéaire, saturée ou insaturée, avantageusement insaturée, préférablement avec un nombre d'insaturations compris entre 1 et 6, et de préférence compris entre 1 et 3 ; ou

des chaînes mono- ou poly- hydroxylées, et/ou mono- ou poly- méthoxylées, et/ou mono- ou poly- oxydées, et/ou mono- ou poly-epoxylées.

Selon un mode de réalisation particulièrement préféré de la présente invention, l'on utilise, à titre de corps gras, une huile, de préférence vierge, sélectionnée parmi l'huile de calophylle inophyle, l'huile de framboise, l'huile de camélia, l'huile d'onagre, l'huile de noix du Brésil, l'huile de baobab et l'huile d'olive, ou un mélange d'au moins deux de ces huiles ; ladite huile, de préférence vierge, étant avantageusement sélectionnée parmi l'huile de calophylle inophyle, l'huile de framboise, l'huile de camélia, l'huile d'onagre, l'huile de noix du Brésil et l'huile de baobab, ou un mélange d'au moins deux de ces huiles ; ladite huile, de préférence vierge, étant préférablement sélectionnée parmi l'huile de

calophylle inophyle, l'huile de framboise, l'huile d'onagre et l'huile de baobab, ou un mélange d'au moins deux de ces huiles ; de manière particulièrement préféré ledit corps gras étant l'huile de calophylle inophyle, de préférence l'huile de calophylle inophyle vierge.

Les susdits diacylglycérols consistent en des 1,2-diacylglycérols et 1,3-diacylglycérols respectivement représentés par les formules (I) et (II) suivantes :

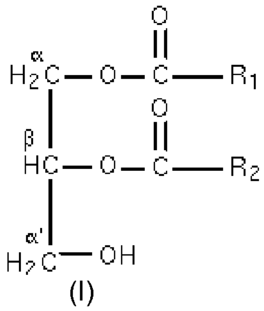
(I)

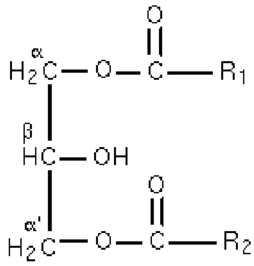
(II)

dans lesquelles R1 et R2 sont :

- des chaînes hydrocarbonées aliphatiques d'un acide gras possédant un nombre de carbone compris entre 12 et 26, avantageusement entre 16 et 20, et de manière particulièrement préférée entre 16 et 18 (en d'autres termes R1 et R2 représentent des chaînes hydrocarbonées aliphatiques dont le nombre de carbone est compris entre 11 et 25, avantageusement entre 15 et 19, et de manière particulièrement préférée entre 15 et 17); ladite chaîne hydrocarbonée aliphatique étant linéaire ou ramifiée, avantageusement linéaire, saturée ou insaturée, avantageusement insaturée, préférablement avec un nombre d'insaturations compris entre 1 et 6, et de préférence compris entre 1 et 3 ; ou
- des chaînes mono- ou poly- hydroxylées, et/ou mono- ou poly- méthoxylées, et/ou mono- ou poly- oxydées, et/ou mono- ou poly-époxylées.

De préférence, lesdits diacylglycérols comprennent majoritairement, et de préférence consistent essentiellement en, des 1,2-diacylglycérols représentés par la formule (I) suivante :

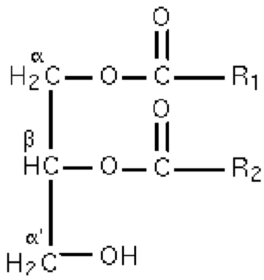

(I)

dans laquelle R1 et R2 sont :

- des chaînes hydrocarbonées aliphatiques d'un acide gras possédant un nombre de carbone compris entre 12 et 26, avantageusement entre 16 et 20, et de manière particulièrement préférée entre 16 et 18 (en d'autres termes R1 et R2 représentent des chaînes hydrocarbonées aliphatiques dont le nombre de carbone est compris entre 11 et 25, avantageusement entre 15 et 19, et de manière particulièrement préférée entre 15 et 17); ladite chaîne hydrocarbonée aliphatique étant linéaire ou ramifiée, avantageusement linéaire, saturée ou insaturée, avantageusement insaturée, préférablement avec un nombre d'insaturations compris entre 1 et 6, et de préférence compris entre 1 et 3 ; ou
- des chaînes mono- ou poly-hydroxylées, et/ou mono- ou poly- méthoxylées, et/ou mono- ou poly- oxydées, et/ou mono- ou poly-époxylées.

L'invention concerne également un procédé de préparation d'une cire enrichie en alcools gras (cire activée), mettant en œuvre le procédé de préparation d'un corps gras enrichi en diacylglycérols susvisé, en effectuant les adaptations suivantes :

- l'on utilise, à titre de corps gras, une cire ; et
- l'on obtient, en lieu et place d'un corps gras enrichi en diacylglycérols, une cire enrichie en alcools gras, avantageusement en alcools gras de formule (III) :

(III) R" - OH

dans laquelle R" représente :

la chaîne hydrocarbonée aliphatique d'un alcool gras comportant de 10 à 34 atomes de carbone, de préférence de 12 à 26 atomes de carbone, et de manière particulièrement préférée de 16 à 22 atomes de carbone ;
ladite chaîne hydrocarbonée aliphatique étant linéaire ou ramifiée, avantageusement linéaire, saturée ou insaturée, préférablement avec maximum de 6 insaturations ; ou
une chaîne mono- ou poly- hydroxylée, et/ou mono- ou poly-méthoxylée, et/ou mono- ou poly-oxydée, et/ou mono- ou poly-époxylée.

En d'autres termes, l'invention concerne un procédé de préparation d'une cire enrichie en alcools gras (cire activée), le dit procédé comprenant les étapes suivantes :

i') mélanger une cire avec de l'eau ou avec une solution saline contenant de préférence un ion bivalent, tel qu'un ion calcium ou magnésium, dans un rapport en volume eau ou solution saline/cire compris entre environ 0,005 et environ 0,5 (de préférence entre 0,005 et 0,5), préférablement entre environ 0,01 et environ 0,4 (de préférence entre 0,01 et 0,4),
ii') maintenir ce mélange sous agitation jusqu'à obtention d'une émulsion entre la cire et l'eau, à une température comprise entre environ 30°C et environ 70°C (de préférence entre 30°C et 70°C), avantageusement d'environ 50°C (de préférence de 50°C), de préférence durant un intervalle de temps compris entre, avantageusement environ 1 et 30 minutes (de préférence entre 1 et 30 minutes), préférablement entre environ 5 et environ 20 minutes (de préférence entre 5 et 20 minutes), et de préférence d'environ 10 minutes (de préférence de 10 minutes),
iii') toujours sous agitation et à ladite température, mettre en présence le mélange obtenu à l'étape précédente avec un volume donné de la fraction non hydrosoluble du suc de *Carica papaya,* enrichie en lipase de *Carica papaya,* obtenue en mettant en œuvre le procédé d'obtention de la fraction non hydrosoluble du suc de *Carica papaya* susmentionné, dans un rapport en volume fraction non hydrosoluble du suc de *Carica papaya*/cire utilisé(e) compris entre environ 0,01 et environ 0,2 (de préférence entre 0,01 et 0,2), avantageusement entre environ 0,05 et 0,015 (de préférence entre 0,05 et 0,015), de préférence d'environ 0,1 (de préférence de 0,1),
iv') maintenir ladite température et l'agitation durant un intervalle de temps compris entre environ une heure et environ six heures (de préférence entre une heure et six heures), avantageusement entre environ deux heures et environ cinq heures (de préférence entre deux heures et cinq heures), de préférence durant environ quatre heures (de préférence durant quatre heures), afin d'obtenir la cire enrichie en alcools gras.

De préférence, ce procédé comprend, après l'étape iv'), l'étape suivante :
v') filtrer le mélange obtenu à l'étape iv'), de préférence en utilisant au moins un filtre à gradient ou au moins une superposition d'au moins deux filtres de porosité décroissante, avantageusement d'environ 500 $\mu$m à environ 250 $\mu$m (de préférence de 500 $\mu$m à 250 $\mu$m), de façon à séparer ladite fraction non hydrosoluble du suc de *Carica papaya,* enrichie en lipase de *Carica papaya,* du milieu réactionnel contenant la cire enrichie en alcools gras.
De préférence, ce procédé comprend après l'étape v'), au moins l'une des deux étapes suivantes, et avantageusement les deux :

- éliminer l'eau résiduelle présente dans le milieu réactionnel contenant la cire enrichie en alcools gras au moyen d'un agent dessiccateur tel que du sulfate de magnésium anhydre, et/ou
- désodoriser et/ou améliorer la brillance de la cire enrichie en alcools gras au moyen d'un charbon actif.

De préférence, au sens de la présente invention, la cire est sélectionnée parmi :

une cire d'origine animale, comme par exemple la cire d'abeille,
une cire d'origine minérale, ou, de préférence,

- une cire d'origine végétale, comme la cire de jojoba, da carnauba, de candelilla, ou leur(s) mélange(s) ;

ladite cire étant avantageusement la cire de jojoba.

De manière particulièrement importante, l'invention a également pour objet un corps gras enrichi en diacylglycérols, susceptible d'être obtenu par le procédé de préparation d'un corps gras enrichi en diacylglycérols selon l'invention (cf. supra). Bien évidemment, l'invention concerne également un corps gras enrichi en diacylglycérols directement obtenu par le procédé de préparation d'un corps gras enrichi en diacylglycérols selon l'invention (cf. supra)

Selon un mode de réalisation préféré, ledit corps gras présente une teneur en diacylglycérols comprise entre environ 5% et environ 30% (de préférence entre 5% et 30%), de préférence entre environ 7% et environ 18% (de préférence entre 7% et 18%), un indice d'acide compris entre environ 15 et environ 50 mg/g (de préférence entre 15 et 50 mg/g) et un indice de peroxyde compris entre environ 5 et 30 mg/g (de préférence entre 5 et 30 mg/g), de préférence inférieur à 20 mg/g.

Ce corps gras enrichi en diacylglycérols est également dénommé « corps gras activé ».

Tel qu'indiqué précédemment, ce corps gras enrichi en diacylglycérols (activé) est une huile d'origine végétale.

De manière particulièrement préférée, ce corps gras enrichi en diacylglycérols (activé) est une huile vierge sélectionnée parmi l'huile de calophylle inophyle, l'huile de framboise, l'huile de camélia, l'huile d'onagre, l'huile de noix du Brésil, l'huile de baobab ou un mélange d'au moins deux de ces huiles ; ladite huile, de préférence vierge, étant avantageusement sélectionnée parmi l'huile de calophylle inophyle, l'huile de framboise, l'huile de camélia, l'huile d'onagre, l'huile de noix du Brésil et l'huile de baobab, ou un mélange d'au moins deux de ces huiles ; de manière particulièrement préféré ledit corps gras étant l'huile de calophylle inophyle, de préférence l'huile de calophylle inophyle vierge. En effet, la Demanderesse a découvert que ces huiles (de préférence vierges) enrichies en diacylglycérols, et de préférence en 1,2 diacylglycérols, présentaient des propriétés cosmétiques particulièrement avantageuses lorsqu'elles sont administrées par application cutanée. C'est pourquoi l'invention s'étend plus largement à une huile enrichie en diacylglycérols (huile activée), avantageusement en 1,2 diacylglycérols, telle que définie précédemment, indépendamment du procédé d'enrichissement en diacylglycérols utilisé pour produire l'huile activée.

Un autre objet de l'invention concerne une cire enrichie en alcools gras (cire activée), susceptible d'être obtenue par le procédé de préparation d'une cire enrichie en alcools gras susvisé. Bien évidemment l'invention concerne également, une cire enrichie en alcools gras (cire activée), directement obtenue par le procédé de préparation d'une cire enrichie en alcools gras susvisé.

L'invention a également pour objet une composition cosmétique comprenant, consistant essentiellement en, ou consistant en :

au moins un corps gras enrichi en diacylglycérols tel que mentionné précédemment en une quantité représentant de 10-6 % à 20 % du poids total de la composition, préférentiellement en une quantité représentant de 10-4 % à 10 %, préférentiellement en une quantité représentant de 10-3 % à 2 %, préférentiellement de 0,01% à 5 %, encore plus préférentiellement de 0,5 à 2,5 % du poids total de la composition finale et

au moins un excipient physiologiquement acceptable.

L'invention a également pour objet une composition cosmétique comprenant, consistant essentiellement en, ou consistant en :

au moins une cire enrichie en alcools gras telle que mentionnée précédemment, en une quantité représentant de 10-6 % à 10 % du poids total de la composition, préférentiellement en une quantité représentant de 10-4 % à 1 % préférentiellement de 0,01% à 3 %, encore plus préférentiellement de 0,1 à 2,0 % du poids total de la composition finale et

au moins un excipient physiologiquement acceptable.

De manière particulièrement importante, l'invention a également pour objet une composition cosmétique comprenant, consistant essentiellement en, ou consistant en, un mélange d'au moins un corps gras enrichi en diacylglycérols tel que mentionné précédemment et d'au moins une cire enrichie en alcools gras telle que mentionnée précédemment, avantageusement dans un rapport en poids cire enrichie en alcools gras/corps gras enrichi en diacylglycérols compris entre 0,01 et 0,11, préférablement entre 0,02 et 0,08, et de préférence d'environ 0,03 (de préférence de 0,03).

L'invention a également pour objet une composition cosmétique comprenant, consistant essentiellement en, ou consistant en :

un mélange d'au moins un corps gras enrichi en diacylglycérols tel que mentionné précédemment et d'au moins une cire enrichie en alcools gras telle que mentionnée précédemment, avantageusement dans un rapport en poids cire enrichie en alcools gras/corps gras enrichi en diacylglycérols compris entre 0,01 et 0,11, préférablement entre 0,02 et 0,08, et de préférence d'environ 0,03 (de préférence de 0,03), et

au moins un excipient physiologiquement acceptable.

L'invention a également pour objet une composition cosmétique comprenant, consistant essentiellement en, ou consistant en :

un mélange d'au moins un corps gras enrichi en diacylglycérols tel que mentionné précédemment et d'au moins une cire enrichie en alcools gras telle que mentionnée précédemment, en une quantité représentant de 10-6 % à 20 % du poids total de la composition, préférentiellement en une quantité représentant de 10-4 % à 10 %, préférentiellement de 0,01% à 5 %, encore plus préférentiellement de 0,5 à 2,5 % du poids total de la composition finale et au moins un excipient physiologiquement acceptable.

La composition selon l'invention pourra être appliquée par toute voie appropriée, notamment orale, ou topique externe, et la formulation des compositions sera adaptée par l'homme du métier.

Préférentiellement, les compositions selon l'invention se présentent sous une forme adaptée à l'application par voie topique. Ces compositions doivent donc contenir un milieu physiologiquement acceptable, c'est-à-dire compatible avec la peau et les phanères, sans risque d'inconfort lors de leur application et couvrent toutes les formes cosmétiques adaptées.

Par application topique, on désigne le fait d'appliquer ou d'étaler la composition selon l'invention, à la surface de la peau ou d'une muqueuse.

Dans le cadre de l'invention, un excipient physiologiquement acceptable désigne un véhicule adapté à une mise en contact avec les couches externes de la peau ou avec les phanères, c'est-à-dire qui ne présente pas de toxicité et ne provoque pas d'irritation, de réponse allergique indue ou encore de réaction d'intolérance. Un excipient physiologiquement acceptable peut comprendre un ou plusieurs composés.

A titre d'excipient physiologiquement acceptable communément utilisé dans le domaine d'application envisagé, on peut citer par exemple des adjuvants nécessaires à la formulation, tels que des solvants, des épaississants, des diluants, des antioxydants, des colorants, des filtres solaires, des agents auto-bronzants, des pigments, des charges, des conservateurs, des parfums, des absorbeurs d'odeur, des huiles essentielles, des vitamines, des acides gras essentiels, des tensioactifs, des polymères filmogènes, etc.

Dans tous les cas, l'homme de métier veillera à ce que ces adjuvants ainsi que leurs proportions soient choisis de telle manière à ne pas nuire aux propriétés avantageuses recherchées de la composition selon l'invention.

Un excipient physiologiquement acceptable peut comprendre un ou plusieurs composés.

Les compositions pour la mise en œuvre de l'invention pourront notamment se présenter sous forme d'une solution aqueuse, hydro-alcoolique ou huileuse, d'une émulsion huile-dans-eau, eau-dans-huile ou émulsions multiples ; elles peuvent aussi se présenter sous forme de suspensions, ou encore poudres, adaptées à une application sur la peau, les muqueuses, les lèvres et/ou les cheveux.

Ces compositions peuvent être plus ou moins fluides et avoir également l'aspect d'une crème, d'une lotion, d'un lait, d'un sérum, d'une pommade, d'un gel, d'une pâte ou d'une mousse. Elles peuvent aussi se présenter sous forme solide, comme un stick ou être appliquées sur la peau sous forme d'aérosol.

A titre d'excipient physiologiquement acceptable communément utilisé dans le domaine d'application envisagé, on peut citer par exemple des adjuvants nécessaires à la formulation, tels que des solvants, des épaississants, des diluants, des antioxydants, des colorants, des filtres solaires, des agents auto-bronzants, des pigments, des charges, des conservateurs, des parfums, des absorbeurs d'odeur, des huiles essentielles, des vitamines, des acides gras essentiels, des tensioactifs, des polymères filmogènes, etc.

Dans tous les cas, l'homme de métier veillera à ce que ces adjuvants ainsi que leurs proportions soient choisis de telle manière à ne pas nuire aux propriétés avantageuses recherchées de la composition selon l'invention. Ces adjuvants peuvent, par exemple, correspondre à 0,01 à 20% du poids total de la composition. Lorsque la composition selon l'invention est une émulsion, la phase grasse peut représenter de 5 à 80% en poids et de préférence de 5 à 50% en poids par rapport au poids total de la composition. Les émulsionnants et co-émulsionnants utilisés dans la composition sont choisis parmi ceux classiquement utilisés dans le domaine considéré. Par exemple, ils peuvent être utilisés en une proportion allant de 0,3 à 30% en poids, par rapport au poids total de la composition.

Selon un autre mode de réalisation avantageux de l'invention, le principe actif selon l'invention peut être encapsulé ou inclus dans un vecteur cosmétique tels que les liposomes ou toute autre nanocapsule ou microcapsule utilisée dans le domaine de la cosmétique ou adsorbé sur des polymères organiques poudreux, des supports minéraux comme les talcs et bentonites.

Avantageusement, la composition selon l'invention peut comprendre, outre l'agent actif selon l'invention, au moins un autre agent actif présentant des effets cosmétiques similaires et/ou complémentaires à ceux de l'invention. Selon l'invention, cet agent actif est défini comme un « agent actif additionnel ».

Par exemple, le ou les agents actifs additionnels peuvent être choisis parmi : les agents anti-âge, raffermissants, éclaircissants, hydratants, drainants, favorisant la microcirculation, agents pharmaceutiques, exfoliants, desquamants, stimulant la matrice extracellulaire, activant le métabolisme énergétique, antibactériens, antifongiques, apaisants, anti-radicalaires, anti-UV, anti-acné, anti-inflammatoires, anesthésiques, procurant une sensation de chaleur, procurant une sensation de fraicheur, amincissants.

De tels agents additionnels peuvent être choisis dans les groupes comprenant :

- la vitamine A et notamment l'acide rétinoïque, le rétinol, le rétinol proprionate, le rétinol palmitate ;
- la vitamine B3 et plus particulièrement le niacinamide, le nicotinate de tocophérol ;
- la vitamine B5, la vitamine B6, la vitamine B12, le panthénol ;
- la vitamine C, notamment l'acide ascorbique, l'ascorbyl glucoside, l'ascorbyl tetrapalmitate, magnésium et sodium ascorbyl phosphate ;
- les vitamines E, F, H, K, PP, le coenzyme Q10 ;
- les inhibiteurs de métalloprotéinase, ou un activateur des TIMP ;
- la DHEA, ses précurseurs et dérivés ;
- les acides aminés tels que l'arginine, ornithine, hydroxyproline, hydroxyproline dipalmitate, palmitoylglycine, hydroxylysine, méthionine et ses dérivés, composés acides aminés N-acyl ;
- les peptides naturels ou de synthèse, incluant les di-, tri-, tetra-, penta- et hexapeptides et leurs dérivés lipophiles, isomères et complexés avec d'autres espèces telles qu'un ion métal (e.g. cuivre, zinc, manganèse, magnésium, et autres). A titre d'exemples, on peut citer les peptides commercialement connus sous le nom MATRIXYL®, ARGIRELINE®, CHRONOGEN™, LAMINIXYL IS™, PEPTIDE Q10™, COLLAXYL ™ (brevet FR2827170, ASHLAND®), PEPTIDE VINCI 01™ (brevet FR2837098, ASHLAND®), PEPTIDE VINCI 02™ (brevet FR2841781, ASHLAND®), ATPeptide™ (brevet FR2846883, ASHLAND®) ou encore le peptide de synthèse de séquence Arg-Gly-Ser-NH2, commercialisé sous le nom ATPeptide™ par ASHLAND®;
- l'extrait d'Artémia salina, commercialisé sous le nom GP4G™ (FR2817748, ASHLAND®);
- les extraits peptidiques végétaux tels que les extraits de lin (Lipigénine™, brevet FR2956818, ASHLAND®), les extraits de soja, d'épeautre, de vigne, de colza, de lin, de riz, de maïs, de pois ;
- les extraits de levures, par exemple le Dynagen™, (brevet FR2951946, ASHLAND®) ou l'Actopontine™ (brevet FR2944526, ASHLAND®) ;
- l'acide déhydroacétique (DHA) ;
- les phystostérols d'origine synthétique ou naturelle ;
- l'acide salicylique et ses dérivés, les alpha- et bêta-hydroxyacides, les silanols ;
- les sucres aminés, glucosamine, D-glucosamine, N-acetyl glucosamine, N-acetyl-D-glucosamine, mannosamine, N-acetyl mannosamine, galactosamine, N-acetyl galactosamine
- les extraits de polyphénols, isoflavones, flavonoïdes, tels que les extraits de raisin, les extraits de pin, les extraits d'olives ;
- les lipides tels que les céramides ou les phospholipides, les huiles d'origine animale, telles que le squalène ou le squalane ; les huiles végétales, telles que l'huile d'amande douce, de coprah, de ricin, de jojoba, d'olive, de colza, d'arachide, de tournesol, de germes de blé, de germes de maïs, de soja, de coton, de luzerne, de pavot, de potiron, d'onagre, de millet, d'orge, de seigle, de carthame, de passiflore, de noisette, de palme, de noyau d'abricot, d'avocat, de calendula ; les huiles végétales éthoxylées, le beurre de karité ;
- tous écrans UV et filtres solaires ;
- l'AMP cyclique et ses dérivés, les agents activateurs de l'enzyme adénylate cyclase et les agents inhibiteurs de l'enzyme phosphodiestérase, l'extrait de centella asiatica, l'asiaticoside et l'acide asiatique, les méthyls xanthines, la théine, la caféine et ses dérivés, la théophylline, la théobromine, la forskoline, l'esculine et l'esculoside, les inhibiteurs d'ACE, le peptide Val-Trp, l'inhibiteurs du neuropeptide Y, l'enkephaline, l'extrait de gingko biloba, l'extrait de dioscorea, la rutine, l'extrait de yerba mate, l'extrait de guarana, les oligosaccharides, les polysaccharides, la carnitine, l'extrait de lierre, l'extrait de fucus, l'extrait hydrolysé de Prunella vulgaris, l'extrait hydrolysé de Celosia cristata, l'extrait d'Anogeissus leiocarpus, l'extrait de feuilles de Manihot utilissima, la palmitoylcarnitine, la carnosine, la taurine, l'extrait de sureau, les extraits d'algue tel que l'extrait de Palmaria Palmata.

L'invention a également pour objet l'utilisation cosmétique d'une quantité efficace d'au moins un corps gras enrichi en diacylglycérols selon l'invention, d'au moins une cire enrichie en alcools gras selon l'invention, ou d'une composition selon l'invention, par application cutanée, pour :

- retarder l'apparition ou limiter les signes du vieillissement cutané, et/ou
- protéger la peau et/ou les phanères contre tous types d'agressions extérieures, et/ou
- favoriser la différenciation épidermique, et/ou
- renforcer la fonction barrière de la peau et/ou des phanères,
- chez un individu humain ou animal (de préférence un mammifère) sain, préférablement humain.

Par « signes du vieillissement cutané », l'on entend toutes modifications de l'aspect extérieur de la peau dues au vieillissement comme, par exemple, les rides et ridules, les défauts pigmentaires tels que les taches de vieillesse ou le manque d'éclat, les poches sous les yeux, les cernes, le flétrissement, la perte d'élasticité, de fermeté et/ou de tonus de la peau, mais également toutes modifications internes de la peau qui ne se traduisent pas systématiquement par un aspect extérieur modifié comme, par exemple, l'amincissement de la peau, ou toutes dégradations internes de la peau consécutives à des stress environnementaux tels que la pollution et les rayonnements UV.

La peau est un organe dont la partie supérieure, le stratum corneum ainsi que le film hydrolipidique qui le recouvre, présente un rôle de barrière physique protectrice vis-à-vis de l'environnement extérieur. Ce rôle de barrière plus communément nommé « fonction barrière de la peau » est d'une importance majeure dans l'homéostasie tissulaire. Or, on sait que des altérations de la fonction barrière résultent des agressions extérieures par des agents tels que les radiations ultraviolettes (UVA et UVB), le froid, la sécheresse, la pollution atmosphérique, certaines substances chimiques, etc. Des altérations de la fonction barrière sont également observées lors du vieillissement de la peau et seraient dues, entre autres, au ralentissement et à des anomalies de la différenciation épidermique, pouvant entrainer une augmentation de la perméabilité cutanée.

La composition selon l'invention permet de lutter contre toutes ces agressions extérieures, en favorisant une différenciation épidermique optimale permettant de maintenir une structure histologique fonctionnelle et une perméabilité physiologique suffisante pour maintenir cette fonction essentielle de barrière que représente la peau.

L'invention a également pour objet un procédé de traitement cosmétique pour retarder l'apparition ou limiter les signes du vieillissement cutané et/ou pour combattre les phénomènes de vieillissement cellulaire au niveau cutané et/ou pour protéger la peau et/ou les phanères contre tous types d'agressions extérieures et/ou pour favoriser la différenciation cellulaire au niveau cutané et/ou pour renforcer la fonction barrière de la peau et/ou des phanères, consistant à appliquer sur au moins une partie de la peau et/ou les phanères une quantité efficace de la composition selon l'invention, telle que définie précédemment.

## Brève description des figures

Dans un souci d'exhaustivité les données expérimentales obtenues par la Demanderesse afin de caractériser la fraction non hydrosoluble du suc de Carica papaya sont présentées ci-après, au sein de la description détaillée de l'invention. Ces données expérimentales se réfèrent aux figures 1-4, lesquelles sont brièvement présentées ci-après :

La figure 1 est un profil électrophorétique des protéines présentes dans le suc de papaye (1), dans la fraction insoluble dans l'eau (également dénommée « non hydrosoluble ») du suc de papaye (2), dans la fraction soluble dans l'eau (également dénommé « hydrosoluble ») du suc de papaye (3), de la papaïne commerciale (Fluka) (4), et des protéines d'un marqueur de poids moléculaire (Invitrogen) (M) sur gel de polyacrylamide 12% ;

La figure 2 est une photographie du Western blot réalisée sur le suc de papaye (1), la fraction insoluble dans l'eau du suc de papaye (2), la fraction soluble dans l'eau du suc de papaye (3) et sur la papaïne commerciale (4) au moyen d'un anticorps dirigé contre la papaïne ;

La figure 3 est gel zymogramme 12% de polyacrylamide et contenant 10% de caséine pour la révélation des protéases ; le gel zymogramme sur lequel ont été déposé du suc de papaye (1), la fraction insoluble dans l'eau du suc de papaye (2), la fraction soluble dans l'eau du suc de papaye (3), la papaïne standard (Sigma, réf P4752) (5) ; ce gel zymogramme étant coloré au bleu de Coomassie ;

La figure 4A est un gel zymogramme en condition non dénaturante sur gel de polyacrylamide 14% révélé au bleu de Coomassie ; gel zymogramme sur lequel ont été déposés du suc de papaye (1), la fraction insoluble dans l'eau du suc de papaye (2), la fraction soluble dans l'eau du suc de papaye (3), la papaïne commerciale (Fluka) (4), ainsi qu'un marqueur de poids moléculaire (M) ;

La figure 4B est un gel zymogramme en condition non dénaturante sur gel de polyacrylamide 14% révélé par recouvrement d'un gel composé d'huile d'olive et de Victoria blue (B) ; gel zymogramme sur lequel ont été déposés du suc de papaye (1), la fraction insoluble dans l'eau du suc de papaye (2), la fraction soluble dans l'eau du suc de papaye (3), la papaïne commerciale (Sigma, réf P4752) (4), ainsi qu'un marqueur de poids moléculaire (M).

La figure 5, quant à elle, est un graphique représentant la quantification du marquage pancytokératine des huiles activées à 0,5% et des huiles non activées de Tamanu, Baobab, Framboise et Onagre dans des échantillons de peau humaine.

## Description détaillée de l'invention

### Définitions

***Corps gras.*** Au sens de la présente invention, un corps gras possède la définition communément admise dans

l'état de la technique, à savoir une substance composée de molécules ayant des propriétés hydrophobes. Les corps gras sont majoritairement composés de triglycérides qui sont des esters issus d'une molécule de glycérol et de trois acides gras. Les autres composants forment ce que l'on appelle la fraction insaponifiable ou « l'insaponifiable ». Les principaux corps gras sont :

- les huiles qui se présentent à l'état liquide à température ambiante car elles sont surtout composées d'acides gras insaturés qui ont des points de fusion faibles ;
- les graisses qui sont pâteuses ou solides à température ambiante car elles sont surtout composées d'acides gras saturés qui ont des points de fusion plus élevés.

Tel qu'indiqué précédemment, à titre de corps gras, l'on utilise, aux fins de la présente invention, une huile d'origine animale, végétale et/ou marine, vierge ou raffinée, avantageusement vierge, ladite huile étant préférablement d'origine végétale et/ou marine, de préférence d'origine végétale.

***Corps gras d'origine marine.*** Les corps gras d'origine marine peuvent être de nature animale, végétale, bactérienne et/ou peuvent être des algues (par exemple des micro-algues, tel qu'indiqué ci-après). A titre d'exemple, l'on peut citer les huiles de poisson, telles que l'huile de foie de requin et de foie de morue, les huiles d'algues et de micro-algues telles que *haemotococcus pluvialis.*

***Huiles d'origine végétale (ou, plus simplement, « huiles végétales »).*** Selon un mode de réalisation particulièrement préféré de l'invention sont utilisées des huiles végétales, telles que l'huile d'olive, l'huile de colza, de soja, de maïs, d'amande douce, d'andiroba, d'argoursier, de babassu, de baies de Laurier, de bourrache, de brocoli, de buriti, de calophylle inophyle, de camélia, de carthame, de cassis, de chanvre, de noix de coco, de pépins de concombre, de cranberry, de pépins de framboise, de fruit de la passion, de pépins de kiwi, de noisettes, de noix du Brésil, d'oléine de karité, de pépins de raisin, de noyaux d'abricots, de sésame, de son de riz, de graines de souchet, pépins de tomate, baobab, onagre, camélia, cameline, chardon-Marie, de pépins de raisin, de carotte, de millepertuis, de graine de lin, de noix, de pépins de grenade, de graines de nigelle, de bourrache, graines de courge, de périlla, de grains de café vert, d'avocat, d'hibiscus, d'argan, de carthame, de rose musquée, de chicouté, de Chia (*salvia hispanica*). Mais aussi de macérât huileux de fleurs telles que la vanille, le millepertuis, le lys, la carotte, bellis, arnica, aloe vera, calendula, figuier de barbarie (liste non exhaustive). Tel qu'indiqué précédemment, parmi ces huiles végétales, sont préférées, pour leurs propriétés cosmétiques particulièrement intéressantes constatées lors de la mise en œuvre de l'invention, l'huile de calophylle inophyle (de préférence vierge), l'huile de framboise (de préférence vierge), l'huile de camélia (de préférence vierge), l'huile d'onagre (de préférence vierge), l'huile de noix du Brésil (de préférence vierge), l'huile de baobab (de préférence vierge) et l'huile d'olive (de préférence) ; étant stipulé que sont particulièrement préférées les six premières.

**Huile de Calophylle Inophyle *(Calophyllum inophyllum L. ; Tamanu).*** Calophyllum, arbre baptisé Tamanu par les Tahitiens et Fohara par les Malgaches, produit une huile remarquable pour son action bénéfique sur la peau. Très aromatique, cette huile est traditionnellement utilisée pour soigner bon nombre d'affections dermatologiques (eczéma, psoriasis, zona...)

De nombreuses études ont démontré que l'huile de Calophylle renfermait de nombreux actifs aux propriétés désinfectantes et protectrices faisant ainsi de cette huile un précieux allié pour les problèmes de peaux.

La fraction saponifiable est composée d'acide linoléique (30-35%), d'acide oléique (30-35%), acide palmitique (15-20%), acide stéarique (15-20%)

La fraction insaponifiable est composée de Inophyllin A : anti-bactérien, action désinfectante ; Calaustraline et inophyllolide : puissants cicatrisants et réparateurs cutanés ; Polyphénols : antioxydants et cicatrisants ils ont aussi une très forte action sur la circulation veineuse ; vitamine E : antioxydant naturel.

**Huile de Baobab *(Adansonia digitata).*** L'huile de Baobab est utilisée dans la pharmacopée sénégalaise pour ses propriétés anti-allergiques et anti-inflammatoires. En cosmétique, cette huile très émolliente et adoucissante est particulièrement efficace pour les peaux sèches, tiraillées et gercées. Réputée pour être cicatrisante et régénérante, elle est conseillée pour le soin des brûlures. La fraction saponifiable est composée d'acide linoléique (22 -26%), acide oléique (30-40%), acide palmitique (20-25%), acide stéarique (2-6%). La fraction insaponifiable est composée Phytostérols (dont beta-sitostérol) et de vitamine E.

**Huile de Noix du Brésil *(Bertholletia excelsa).*** La Noix du Brésil ou Noix d'Amazonie est issue d'un arbre sud-américain appelé : Bertholletia excelsa. Source végétale de phospholipides, de tocophérols et de phytostérols, l'huile de noix du Brésil apporte douceur, confort et élasticité à la peau tout en jouant son rôle d'antioxydant naturel.

La fraction saponifiable est composée d'acide linoléique (40-45%), acide oléique (oméga-9) (30-35%), acide palmitique (10-15%), acide stéarique (5-10%). La fraction insaponifiable est composée de phytostérols (dont beta-sitostérol) : ils maintiennent la structure et la fonction de la membrane cellulaire et réduisent les inflammations ; Squalènes : principaux composants de la surface de la peau, ils présentent des propriétés émollientes et antioxydantes., Tocophérols : vitamine E naturelle, antioxydants puissants qui préviennent le rancissement des acides gras et protègent les cellules des dommages liés aux radicaux libres ; Phospholipides : composés similaires à ceux qui forment les membranes cellulaires, propriétés émollientes et protectrices ; Sélénium : oligo-élément aux vertus antioxydantes.

**Huile d'Onagre (*Oenothera* biennis).** L'huile d'Onagre est très riche en acide linoléique et c'est une des rares huiles à contenir l'acide gamma-linolénique. Ces acides gras essentiels, reconstructeurs des membranes cellulaires, possèdent des propriétés adoucissantes, revitalisantes, restructurantes et anti-rides exceptionnelles. Egalement riche en vitamine E, l'huile d'onagre préserve la peau d'un vieillissement précoce.

La fraction saponifiable est composée d'acide linoléique (70-75%), gamma-linolénique (5-10%) acide oléique (4-8%), acide palmitique (4-8%), acide stéarique (1-4%)

La fraction insaponifiable (1,5 à 2%) est composée de phytostérols : action cicatrisante et réparatrice, ils réduisent également les inflammations ; triterpènes : action anti-radicalaire, ils protègent les tissus de la dégénérescence ; Stérols dont de la vitamine E : antioxydants naturels.

**Huile de Camélia *(Camellia sinensis L.).*** L'huile de camélia, appelée également huile de thé vert est extraite des graines du théier. L'huile végétale de camélia possède des propriétés nourrissantes, protectrices, adoucissantes, assouplissantes pour la peau.

La fraction saponifiable est composée d'acide linoléique (5-10%), acide oléique (75-80%), acide palmitique (5-10%), acide stéarique (1-5%).

La fraction insaponifiable (1,5 à 2%) est composée de triterpènes ; squalane ; saponines ainsi que du kaempférol et ses glycosides : ce sont des composés fortement anti-inflammatoires, comme la quercétine. Ils aident à protéger le corps contre les dommages causés par des conditions inflammatoires.

**Huile de Framboise *(Rubus idaeus L.).*** L'huile de Framboise possède des propriétés cicatrisantes grâce à sa teneur exceptionnelle en acides gras essentiels (oméga-3 et oméga-6), elle s'utilise également pour soulager les démangeaisons et l'eczéma. L'huile de framboise est aussi connue pour absorber une partie des rayons UVA et UVB, apportant ainsi une protection légère au soleil. Sa grande richesse en antioxydants et en caroténoïdes en fait aussi une huile idéale pour les soins préparateurs au soleil et réparateurs après soleil.

La fraction saponifiable est composée d'acide linoléique (50 -55 %) et acide alpha-linolénique (30-35%), acide oléique (oméga-9) (10-15 %) acide palmitique (0-5%)

La fraction insaponifiable est composée de vitamine E (environ 5600 mg/kg) : antioxydant naturel ; d'acide gallique : antioxydant naturel ; de caroténoïdes (bêta-carotène, lutéine, et cryptoxanthine) : action anti-radicalaire, ils protègent les tissus de la dégénérescence.

**Huile d'Olive** (***Olea europaea L.***). L'olivier est connu depuis des millénaires pour sa longévité exceptionnelle. C'est d'ailleurs le premier arbre qui soit cité dans l'histoire du monde.

Obtenue par pression à froid de sa pulpe et non de ses noyaux, l'huile d'olive est une des huiles les plus riches en acide oléique. Nourrissante, adoucissante et émolliente, on retrouve l'huile d'Olive dans la composition des traditionnels savons d'Alep et de Marseille.

Excellent agent cicatrisant, elle est dotée d'une concentration non négligeable en insaponifiables (environ 1 à 2 %) qui lui offrent des vertus antioxydantes, apaisantes et protectrices face aux méfaits du temps et du soleil. En interne, l'huile d'olive possède des propriétés digestives et légèrement laxatives qui lui vaut d'être conseillée dans les troubles digestifs et notamment ceux de l'estomac.

La fraction saponifiable est composée d'acide linoléique (15-20%), acide oléique (55-60%), acide palmitique (15-20%), acide stéarique (0-5%)

La fraction insaponifiable est composée de composés phénoliques (hydroxytyrosol) : antioxydants majoritaires ; phytostérols : action cicatrisante et réparatrice ; squalènes : principaux composants de la surface de la peau, ils présentent des propriétés émollientes et antioxydantes, Vitamine E (alpha-tocophérol), Chlorophylle : antioxydants naturels.

Lorsque le corps gras utilisé aux fins de la présente invention est une huile (mode de réalisation préféré), cette huile peut être vierge ou raffinée. Selon un mode de réalisation particulièrement préféré de l'invention, l'on utilise préférentiellement au moins une huile vierge, et de préférence une huile végétale vierge. En effet, la plateforme technologique développée par la Demanderesse présente l'avantage - significatif - de pouvoir obtenir des huiles vierges, de préférence des huiles végétales vierges, enrichies en diacylglycérols et/ou en alcools gras (principes actifs cosmétiques) sans imposer de recourir à des huiles raffinées.

***Huiles végétales vierges.*** Les huiles végétales dites « vierges » sont des huiles végétales pures, extraites uniquement par des procédés mécaniques de fruits, graines, noyaux, généralement par première expression à froid (à une température maximale de 60°C) à partir d'une plante cultivée. Ces huiles sont caractérisées par leur fraction insaponifiable et saponifiable. La fraction insaponifiable est la fraction résiduelle qui est insoluble dans l'eau mais soluble dans les acides organiques après saponification. La teneur en insaponifiable des matières grasses est généralement de l'ordre de 0,5 à 2 %. Il s'agit d'un mélange complexe comprenant des stérols, des hydrocarbures (squalène, ...), des triterpènes, des alcools gras (cires), des pigments liposolubles, des vitamines, etc. La fraction insaponifiable des huiles végétales trouve des applications en cosmétique pour ses propriétés biologiques. La fraction saponfiable des huiles est caractérisée par les acides gras, glycérides et tricycérides. Les acides gras sont de deux types, Acides gras saturés, Acides gras insaturés (mono-insaturés/AGMI ou poly-insaturés/AGPI). L'utilisation de ces huiles végétales vierges, en

tant que corps gras, est particulièrement préférée au sens de la présente invention.

***Huiles raffinées.*** Même si, tel qu'indiqué précédemment, l'utilisation d'une huile vierge, et de préférence d'une huile végétale vierge, est privilégiée au sens de la présente invention, cette dernière fonctionne également très bien avec des huiles raffinées. Pour qu'une huile soit raffinée, elle doit subir une pression à chaud. Avec un pressage qui s'effectue à une température se situant entre 80°C et 120°C. On obtient alors une huile brute, qui ne peut être consommée telle quelle, elle doit alors subir une longue série de traitements dans le but d'éliminer les goûts et couleurs indésirables. L'étape de dégommage qui consiste à retirer les substances qui contribuent à l'instabilité et à la production de mousse et de fumée lors des fritures (acides gras libres, phospholipides), par brassage, avec de l'eau acidulée, qui va hydrolyser et ainsi séparer les substances. Ensuite le goût est neutralisé avec une solution de soude. Vient ensuite une décoloration par brassage à 90°C avec de la terre décolorante et un filtrage qui permettent de débarrasser l'huile de ses pigments. Le processus se termine par une désodorisation à l'aide de vapeur d'eau sous faible pression pour mieux conserver les qualités de l'huile. On obtient une huile raffinée, claire, inodore et faible en saveurs. Ces huiles sont uniquement caractérisées par leur fraction saponifiable, la fraction insaponifiable étant éliminée par les traitements.

La Demanderesse a découvert que les composants de la fraction insaponifiable, à savoir des stérols, des hydrocarbures (squalène, etc.), des triterpènes, des alcools gras (cires), des pigments liposolubles, des vitamines (liste non exhaustive) permettaient d'augmenter l'activité biologique (cosmétique) des corps gras activés et compositions les contenant selon la présente invention. C'est une des raisons pour lesquelles l'emploi d'une huile vierge (de préférence d'une huile végétale vierge), comprenant cette précieuse fraction insaponifiable, est particulièrement préféré en ce qui concerne la présente invention, la plateforme technologique constituant la présente invention étant tout à fait adaptée à l'enrichissement/activation d'une huile vierge.

***Corps gras activé / huile activée.*** La présente invention vise notamment à obtenir un corps gras (de préférence une huile) enrichi en diacylglycérols, en particulier en 1,2 diacylglycérols ; les diacylglycérols présents au sein du corps gras/de l'huile ainsi enrichi(e) jouant le rôle de principes actifs cosmétiques, dans le cadre d'une application cutanée. En d'autres termes, les corps gras enrichis en diacylglycérols ont une structure modifiée et une activité biologique (cosmétique) accrue au niveau cutané. C'est pourquoi les corps gras / huiles ainsi enrichi(e)s sont également dénommé(e)s corps gras activés / huiles activées.

***Cire.*** Une cire est un ester de l'éthylène glycol et de deux acides gras ou un monoester d'acide gras et d'alcool à longues chaînes. Les cires pouvant être d'origine animale, comme par exemple la cire d'abeille, d'origine végétale, comme le cire de jojoba, de carnauba, de candelilla, ou encore d'origine minérale.

Les alcools gras sont des alcools aliphatiques à longue chaîne hydrocarbonée présentant une seule fonction hydroxyle en position terminale. Par un alcool gras, on entend les composés de formule (III) :

(III) $R''-OH$

dans laquelle R" représente la chaîne aliphatique d'un alcool gras. Cet alcool gras étant constitué par une chaîne carbonée comportant de 10 à 34 atomes de carbone.

Selon un mode de réalisation particulier de l'invention, les alcools gras possèdent une chaîne carbonée dont le nombre de carbone est compris entre 12 à 26. Selon un autre mode de réalisation préféré de l'invention, l'alcool gras possède sur sa chaîne aliphatique entre 16 et 22 atomes de carbone.

Ainsi, ces alcools gras seront, de manière préférentielle, choisis parmi le groupe constitué d'alcool gras à chaîne aliphatique de taille moyenne, saturée ou insaturée.

Par ailleurs, la chaîne aliphatique des alcools gras, c'est-à-dire le groupement R", peut être une chaîne carbonée linéaire ou ramifiée, et/ou saturée ou insaturée, le nombre d'insaturation étant compris entre 1 et 6. Le groupement R" peut être aussi une chaîne mono- ou poly- hydroxylée, et/ou mono- ou poly-méthoxylée, et/ou mono- ou poly-oxydée, et/ou mono- ou poly-époxylée. Elle pourra ainsi être présente sous toutes les formes naturelles existantes.

Ces alcools gras se trouvent en grande quantité dans les cires. Ces cires pouvant être d'origine animale, comme par exemple la cire d'abeille, d'origine végétale, comme la cire de jojoba, de carnauba, de candellila, ou encore d'origine minérale.

Ainsi, par exemple, l'hydrolyse contrôlée de la cire de l'huile de jojoba (abrégée en « cire de jojoba » ; cire qui constitue plus de 96 % de l'huile de jojoba) a pour effet de libérer les principaux alcools gras aliphatiques contenus dans celle-ci, ce qui permet d'obtenir un mélange d'alcools gras, à chaîne hydrocarbonée linéaire saturée ou insaturée.

En particulier, l'hydrolyse de la cire de l'huile de jojoba permet d'obtenir un mélange d'alcools gras d'un haut degré de pureté comportant, dans leur chaîne aliphatique, entre 16 et 22 atomes de carbone.

La cire de l'huile de jojoba est la cire liquide contenue dans la graine de Jojoba (Simmondsia chinensis), plante buissonnante originaire du sud de l'Arizona et de la Californie, ainsi que du nord-ouest du Mexique. Les graines de jojoba ont un rendement en huile d'environ 50% de leur poids. L'huile de jojoba est un mélange d'esters céridiques, possédant des chaînes de 36 à 46 atomes de carbone. Chaque molécule se compose d'un acide gras et d'un alcool gras liés par une liaison ester. L'huile de jojoba est caractérisé par la présence de 10% d'acide oléique ($C_{18:1}$), 70%

d'acide gadoléique ($C_{20:1}$), 15% d'acide érucique ($C_{22:1}$), 5% d'acide nervonique ($C_{24:1}$) et des alcools gras associés, octacosénol ($C_{18:1}$), eicosénol ($C_{20:1}$), docosénol ($C_{22:1}$) et tétracosénol($C_{24:1}$). Ainsi l'hydrolyse contrôlée de la cire de jojoba a pour effet de libérer les principaux alcools gras aliphatiques contenus dans celle-ci, ce qui permet d'obtenir un mélange d'alcools gras, à chaîne linéaire saturée ou insaturée.

Ainsi par exemple, l'hydrolyse contrôlée de la cire de jojoba (cire qui constitue plus de 96% de l'huile de jojoba) a pour effet de libérer les principaux alcools gras et acides gras aliphatiques contenus dans celle-ci, ce qui permet d'obtenir une cire enrichie en alcools gras et acides gras à chaîne linéaire hydrocarbonée saturée et insaturée. En particulier, l'hydrolyse de l'huile de jojoba par la présente invention permet d'obtenir une cire enrichie en alcools gras et acides gras comportant entre 18 et 24 atomes de carbone. Cette hydrolyse permet ainsi de concentrer tous les principes actifs qui sont susceptibles d'agir sur la peau.

Le mélange d'acides gras et d'alcools gras selon l'invention sera préférentiellement obtenu à partir d'une hydrolyse des cires présentes dans l'huile de jojoba (Simmondsia chinensis). Cette hydrolyse permettant de libérer les alcools gras constitutifs de cette cire. Cette hydrolyse peut être réalisée de manière enzymatique en utilisant une triacylglycérol hydrolase dont l'activité principale est d'hydrolyser la liaison ester afin de libérer un alcool et un acide. Une des possibilités particulièrement avantageuses de cette invention est d'utiliser la même lipase que celle utilisée pour l'hydrolyse des triacylglycérols.

Préférentiellement selon l'invention, les actifs constituant l'association c'est-à-dire l'huile enrichie en diacylglycérols et une cire enrichie en acides gras et alcools gras, seront d'origine végétale, marine ou animale.

***Cire activée.*** La présente invention vise notamment à obtenir une cire (de préférence la cire de l'huile de jojoba, abrégée en « cire de jojoba ») enrichie en alcools gras, tels que définis précédemment. Les alcools gras présents au sein de la cire ainsi enrichie jouent le rôle de principes actifs cosmétiques, avantageusement en combinaison avec les diacylglycérols du corps gras/de l'huile activé(e) (cf. supra), dans le cadre d'une application cutanée. En d'autres termes, la cire enrichie en alcools gras présente une structure modifiée et une activité biologique (cosmétique) accrue au niveau cutané. C'est pourquoi la cire ainsi enrichie est également dénommée cire activée.

***Lipase de Carica papaya.*** Les lipases (triacylglycérol hydrolase ; EC.3.1.1.3) sont des enzymes réversibles qui, dans le sens le plus favorable de réaction, hydrolysent les esters du glycérol. On peut classer ces enzymes en plusieurs groupes, en fonction de leurs différentes spécificités : spécificité vis-à-vis du substrat ; spécificité de position ou stéréospécificité ; spécificité vis-à-vis de la nature des acides gras ou typosélectivité ; spécificité vis-à-vis d'une position ou stéréospécificité Dans la présente invention, l'on utilise une lipase stéréospécifique de type sn3. Cette lipase hydrolyse ainsi les triglycérides en formant préférentiellement du 1-2, diacylglycérol. La lipase utilisée peut être d'origine végétale (exemple : Carica *papaya*) ou microbienne (exemple : Penicelium cyclopium). La plateforme technologique développée par la Demanderesse est basée sur l'utilisation de la lipase de *Carica papaya* (Villeneuve et al. JAOCS, 72, 6:753.1995).

La Papaye (*Carica papaya*) est cultivée principalement dans les régions tropicales et subtropicales du monde entier. La papaye est un membre de la famille des *Caricaceae,* qui appartient à famille des Brassicales. Le latex de *Carica papaya* est déjà connu pour ses endopeptidases, une source riche en cystéine y compris la papaïne, la chymopapaïne et caricain. Ces protéinases peuvent être extraites sous forme de protéines du latex soluble dans l'eau. La présence de l'activité lipase a été rapportée par Giordani et al. Jusqu'à récemment, toutes les tentatives pour solubiliser l'activité enzymatique à partir de cette fraction de latex ont échoué. La lipase de *Carica papaya* latex (CPL) a donc été traditionnellement considérée comme un biocatalyseur "naturellement immobilisé". Une poudre sèche contenant l'activité de la lipase peut être obtenue après le lavage des particules de latex avec l'eau et les centrifugations. La stéréospécificité et typo-sélectivité du suc brut *Carica papaya* latex ont été étudiées à la fois pendant l'hydrolyse et lors des réactions acyltransfer. Au cours du processus d'hydrolyse, une activité 1,3-stéréospécifique a été déterminé par ce biocatalyseur, avec une activité sn-3 stéréo de préférence.

Dans le cadre de ses démarches expérimentales, la Demanderesse a mis en évidence une certaine typosélectivité de cette lipase pour les acides gras, la présente invention, dans les conditions expérimentales décrites, orientant celle-ci vers des triacylglycérols possédant des acides gras à longue chaîne, de longueur comprise entre 18 et 20, et présentant entre aucune et 3 insaturations. En effet, des essais réalisés préalablement ont montré que dans les conditions opératoires utilisées lors de l'invention, les rendements d'obtention d'une huile enrichie en 1,2-diacylglycérols sont plus importants avec une huile possédant majoritairement des acides gras de longueur de chaîne compris entre 18 et 20 carbones et possédant des insaturations (huile d'olive par exemple) qu'avec une huile dont les acides gras saturés possèdent une longueur de chaîne comprise respectivement entre 8 et 10 carbones (caprylic/capric triglycérides - Mygliol ® 812), 12 et 16 carbones (beurre animal), 16 et 18 carbones (huile de palme). Les résultats sont résumés dans le tableau 1 infra.

Tableau 1 : comparaison des rendements d'enrichissement en DAG en utilisant la lipase de *Carica papaya* à partir de différents corps gras

| Huile enrichie | Huile d'olive | Myglyol 812 | Beurre (lait) | Huile de palme |
|---|---|---|---|---|
| Indice d'acide (mg/g) | 17.5 | 10.2 | 3.2 | 13.6 |
| Teneur en 1,2-diacylglycér ols (%) | 15 | 6.8 | 1 | 8.9 |
| Teneur en 1,3-diacylglycér ols | 3 | 2 | 0 | 3.2 |

Tel qu'indiqué précédemment, la plateforme technologique développée par la Demanderesse est basée sur l'utilisation de la lipase de Carica papaya, et plus particulièrement sur l'utilisation de la fraction non hydrosoluble de son suc. Toutefois, la présente invention s'étend, de manière plus large, également à un corps gras enrichi en diacylglycérols, au moyen de toute lipase adaptée (de préférence au moyen d'une lipase stéréospécifique de type sn3, par exemple d'origine végétale ou microbienne), avantageusement en 1,2-diacylglycérols, de préférence en 1,2-diacylglycérols de formule (I) tels que définis précédemment, ledit corps gras étant sélectionné parmi l'huile (de préférence vierge) de calophylle inophyle, l'huile (de préférence vierge) de framboise, l'huile (de préférence vierge) de camélia, l'huile (de préférence vierge) d'onagre, l'huile (de préférence vierge) de noix du Brésil et l'huile (de préférence vierge) de baobab, ledit corps gras étant l'huile (de préférence vierge) de calophylle inophyle. En outre, l'invention s'étend également à une composition comprenant, consistant essentiellement en, ou consistant en, ledit corps gras enrichi en diacylglycérols (avantageusement en 1,2-diacylglycérols, de préférence en 1,2-diacylglycérols de formule (I) tels que définis précédemment) et une cire enrichie en alcools gras (de préférence de formule (III) tels que définis précédemment).

***Suc de Carica papaya (également dénommé : « suc brut de Carica papaya »)***. Le suc de papaye est obtenu à partir du Latex de *Carica papaya.* Le latex est recueilli en incisant les fruits encore verts. On entaille superficiellement le fruit vert pour recueillir le latex blanc qui se fige au contact de l'air. Il se compose principalement de protéines dont des enzymes endopeptidases (Papaïne brute = papaïne, chymopapaïnes, papayaprotéinase ; la papaïne étant une protéine de 212 acides aminés pour une masse moléculaire de 23000 daltons), une lipase (lipase de *Carica papaya,* dénommée *Carica papaya* lipase, en langue anglaise; cf. Villeneuve et al. JAOCS, 72, 6:753.1995), ainsi que de sucres et de vitamines.

***Phanères.*** Les phanères sont des productions protectrices apparentes de l'épiderme, caractérisées par une kératinisation intense. Les cheveux, les dents, les ongles et les poils sont des phanères.

***Peau.*** Au sens de la présente invention, la peau s'entend, au sens large, de l'organe constituant le revêtement du corps et inclut notamment le cuir chevelu (peau du crâne recouverte de cheveux).

Caractérisation du suc de papaye purifié, et plus particulièrement de la fraction insoluble dans l'eau (non hydrosoluble) de ce suc de papaye

Lors de la mise en suspension dans l'eau du suc de papaye séché, les enzymes présentant les activités lipolytiques et protéasiques ont présenté des solubilités différentes : les protéases étant solubles contrairement aux enzymes responsables de l'activité lipase *(Giordani et al., 1991).* Cette propriété a permis de séparer partiellement le suc de papaye en deux fractions (cf. Tableau 2 infra).

Tableau 2 : Etude et caractérisation du fractionnement du suc de papaye

| | Matières sèches Proportion | Quantité totale / fraction | Protéines Pourcentage / matière sèche | Quantité totale / fraction |
|---|---|---|---|---|
| Suc de papaye brut | 82 % | 82 % | 78 % | 64,5 % |
| Fraction insoluble | 19 % | 17% | 47 % | 8% |
| Fraction soluble | 7% | 59 % | 95 % | 56 % |

Le fractionnement du suc de papaye a été caractérisé par la teneur en matières sèches ainsi qu'en protéines des fractions obtenues, ces valeurs sont comparées à celles du suc. Le suc utilisé possède 18% d'humidité, sa mise en suspension à 10% dans l'eau ainsi que la séparation des deux fractions par centrifugation entraîne la récupération de 92% de la matière sèche de départ et 99,7% des protéines initialement présentes. Parmi ces protéines, 88% se

retrouvent dans la fraction soluble dans l'eau et 12% dans la fraction particulaire insoluble dans l'eau (cf. Tableau 2 supra). Dans un second temps, les protéines obtenues par le procédé de séparation ont été caractérisées plus précisément. Ainsi, après étude des activités enzymatiques protéases et lipases, il a pu être déterminé le poids moléculaire des protéines présentes dans ces deux fractions. La quantité de protéines présentes dans le suc brut ainsi que dans chacune des fractions a été déterminée selon la méthode de Kjeldahl et est reportée dans le Tableau 3 infra. L'activité protéasique et l'activité lipolytique ont ensuite été mesurées pour le suc de papaye ainsi que dans les fractions soluble et insoluble dans l'eau.

Tableau 3 : Activités enzymatiques spécifiques et globales du suc de papaye ainsi que des fractions solubles et insolubles dans l'eau

| | **Activité protéolytique** | | | **Activité lipolytique** | | |
|---|---|---|---|---|---|---|
| | **Suc de papaye brut** | **Fraction soluble** | **Fraction insoluble** | **Suc de papaye brut** | **Fraction soluble** | **Fraction insoluble** |
| **Activité enzymatique (U/mg matière sèche)** | 1,5 | 1,35 | 0,1 | 0,67 | *indétectable* | 0,66 |
| **Activité enzymatique spécifique (U/mg protéine)** | 2,35 | 2,4 | 1,3 | 1,05 | / | 7,45 |
| **Facteur de purification** | / | **1,02** | / | / | / | **7,1** |
| **Activité enzymatique totale** | **150** | **134** | **11** | **67** | / | **60** |

La mise en suspension dans l'eau du suc de papaye permet de séparer, de manière efficace, l'activité protéase de l'activité lipase contenue initialement dans le suc. En effet, environ 90% de l'activité protéolytique et de l'activité lipolytique présentes initialement se retrouvent respectivement dans les fractions solubles et insolubles dans l'eau.

Le poids moléculaire des protéines présentes dans le suc de papaye et dont les fractions solubles et insolubles dans l'eau ont été déterminées sur gel de polyacrylamide à 12% en conditions dénaturantes et réductrices. Le profil électrophorétique ainsi obtenu est représenté en figure 1. La piste M correspond à un marqueur de poids moléculaire (Invitrogen), la piste 1 correspond au suc de papaye, la piste 2 correspond à la fraction insoluble dans l'eau du suc de papaye, la piste 3 correspond à la fraction soluble dans l'eau de ce suc de papaye et la piste 4 correspond à la papaïne achetée dans le commerce (Fluka).

Les poids moléculaires des principales protéines observées ont été déterminés par corrélation avec celles du marqueur de poids moléculaires en utilisant la représentation log PM = f(Rf) (Rf = distance de migration de protéine / distance de migration du front du gel).

Ces poids moléculaires sont présentés ci-après, au sein du tableau 4.

Tableau 4 : Poids moléculaires des protéines présentes dans le suc de papaye, les fractions insolubles et solubles dans l'eau, ainsi que dans la papaïne commerciale.

| | **Suc de papaye brut séché** | **Fraction insoluble** | **Fraction soluble** | **Papaïne purifiée commerciale** |
|---|---|---|---|---|
| **Poids molécul aire des protéin es (kDa)** | 35<br><br>23<br>22<br>18 | 23 | 35<br>28<br>23<br>22<br>18<br>15 | 35<br><br>23<br>22<br>18<br>15 |

Le suc de papaye comporte quatre protéines majoritaires de poids moléculaires respectifs de 18, 22, 23 et 35 kDa. La fraction insoluble dans l'eau paraît être composée d'une seule protéine de poids moléculaire de 23 kDa. La fraction soluble dans l'eau de six protéines de poids moléculaire de 15, 18, 22, 23, 28 et 35 kDa, la présence d'une protéine de 15 kDa doit être dûe à une hydrolyse survenue lors de la mise en suspension du suc de papaye. La papaïne commerciale, qui ne comporte pas d'activité lipolytique est composée des cinq mêmes formes protéiques que le suc

de papaye. La similitude des profils électrophorétiques est certainement due au fait que cet extrait commercial est un mélange d'endopeptidases du suc de papaye sous la forme de proenzymes et/ou d'enzymes matures.

D'après ces résultats, la lipase de *Carica papaya* semble posséder un poids moléculaire de 23 kDa. Cependant, une protéine de même poids moléculaire est également présente dans la fraction soluble dans l'eau et la papaïne commerciale dans lesquelles une seule activité protéasique a été révélée.

*Caractérisation par western blot*

Une analyse de ces mêmes échantillons par un western blot a ensuite été réalisée afin d'identifier le poids moléculaire des protéines correspondant aux activités de type papaïne.

L'anticorps utilisé, un anticorps polyclonal issu de sérum caprin dirigé contre la papaïne (Rockland, anti-Papain [*Carica papaya*]) révèle principalement une protéine de 35 kDa. Des signaux plus faibles sont observés pour des protéines de poids moléculaire compris entre 15 et 28 kDa.

La papaïne est décrite pour posséder un poids moléculaire pouvant varier de 21 à 23 kDa (*Azarkan et al., 2003*). L'anticorps utilisé s'est apparemment fixé sur la propapaïne, proenzyme précurseur de la papaïne possédant une région N-terminale (134 acides aminés) suivie de l'enzyme mature composée de 212 acides aminés. En effet, cette proenzyme est constituée de 345 acides aminés (*Taylor et al., 1999* ; *Azarkan et al., 2003*).

L'analyse plus précise des signaux de faible intensité permet d'identifier la présence d'une fixation moins spécifique de l'anticorps sur des protéines de poids moléculaire 28 kDa pour les fractions insoluble et insoluble dans l'eau ainsi que pour une protéine de 22 kDa pour la fraction soluble dans l'eau.

Pour la fraction insoluble dans l'eau, l'anticorps anti-papaïne ne s'est pas fixé sur la protéine de poids moléculaire 23 kDa. Cela confirme donc que cette protéine n'est pas une protéase de type papaïne mais bien la lipase de *Carica papaya.*

*Caractérisation par gel zymogramme*

La technique de gel zymogramme permet de déterminer la présence d'enzymes dans un mélange de protéines en mettant en évidence leur activité propre. Un substrat spécifique est préalablement inclus dans le gel de polyacrylamide, les enzymes formant des pistes distinctes et révélées par la même réaction sont appelées isozymes.

La mise en œuvre de deux gels zymogrammes (lipolytique et protéolytique) a été envisagée afin de localiser la présence des protéases et de la lipase de *Carica papaya* dans les différents extraits.

- Zymogramme pour la révélation des protéases

Le gel zymogramme Novex casein zymogram utilisé est composé d'un gel de séparation composé de 12% de polyacrylamide et contenant 10% de caséine, substrat potentiel de protéases. Ce gel est réalisé en conditions non réductrices afin de conserver les activités enzymatiques. Les enzymes possédant une activité protéolytique vont hydrolyser localement la caséine du gel. Après coloration du gel au bleu de Coomassie, les zones où les protéines ont été hydrolysées sont mises en évidence par leur couleur blanche sur fond bleu uniforme, comme représenté en figure 3. Sur cette figure 3, la piste 1 correspond au suc de papaye, la piste 2 correspond à la fraction insoluble dans l'eau du suc de papaye, la piste 3 correspond à la fraction soluble dans l'eau du suc de papaye et la piste 4 correspond à de la papaïne achetée dans le commerce (Fluka).

L'hydrolyse de la caséine, et donc la présence d'une activité protéase plus ou moins importante, est observée, pour toutes les fractions analysées (Figure 3). Deux isoformes sont observés, l'isoforme 2 possédant une intensité plus importante pour la fraction soluble dans l'eau et la papaïne commerciale.

- Zymogramme pour la révélation des lipases

La réalisation de zymogrammes permettant de déceler une activité enzymatique de type lipase est plus complexe que pour les protéases. La littérature décrit un système constitué de deux gels, un premier dans lequel les protéines sont séparées et un deuxième contenant le substrat (*Gilbert et al., 1991 ; Abousalham et al., 2000*). Dans un premier temps, les protéines migrent sur un gel de polyacrylamide en condition native. Ensuite, la présence de lipases est révélée par application d'une surcouche composée d'un deuxième gel de polyacrylamide contenant à la fois le substrat lipidique et un indicateur coloré, le Victoria blue, dont la couleur est bleue en milieu acide et rouge en milieu basique (*Yadav et al., 1997).* Dans ce type de zymogramme, la présence d'une activité lipasique est donc visualisée par l'apparition d'une coloration bleue correspondant aux zones d'hydrolyse des triacylglycérols de l'huile d'olive, et donc à la production d'acide oléique.

Deux gels de polyacrylamide sont réalisés en parallèle. Un premier gel témoin est coloré à l'aide du bleu de

Coomassie (Figure 4A) et permet de visualiser le profil de migration des protéines en condition non dénaturante. En parallèle, un deuxième gel zymogramme est révélé avec la surcouche contenant le substrat (cf. Figure 4B).

De manière plus spécifique, la figure 4A représente un gel zymogramme en condition non dénaturante sur gel polyacrylamide 14 % révélé au bleu de Coomassie et la figure 4B représente un gel zymogramme en condition non dénaturante sur gel polyacrylamide 14 % révélé par recouvrement d'un gel composé d'huile d'olive et Victoria blue (B).

Les pistes pour les gels zymogrammes objets des figures 4A et 4B sont identiques et correspondent :

- au suc de papaye pour ce qui concerne la piste 1,
- à la fraction insoluble dans l'eau du suc de papaye pour ce qui concerne la piste 2,
- à la fraction soluble dans l'eau du suc de papaye pour ce qui concerne la piste 3,
- à de la papaïne achetée dans le commerce (Fluka) pour ce qui concerne la piste 4, et
- à un marqueur de poids moléculaire pour ce qui concerne la piste (M).

En condition non dénaturante, la migration n'est pas précise et seules deux entités protéiques sont observées. (Figure 4A).

En ce qui concerne le zymogramme proprement dit, une coloration bleue du gel correspondant à une production d'acide oléique est observée pour le suc de papaye ainsi que pour sa fraction insoluble dans l'eau. L'activité lipolytique n'est donc présente que dans ces fractions.

La papaïne commerciale ne présente pas d'activité lipase. La protéine de 23 kDa présente dans cet extrait ne correspond donc pas à une lipase comme l'étude des activités enzymatiques des différentes fractions l'avait déjà suggéré (cf. figure 2 supra).

Cette étude par zymogramme permettant de révéler une activité protéolytique et lipolytique a permis de confirmer que les endopeptidases de la papaye sont un groupement de protéines présentant une activité enzymatique protéolytique importante dans les extraits étudiés.

Cette étude a permis également de démontrer que seul le suc de papaye et sa fraction insoluble dans l'eau possèdent une activité lipolytique qui est liée à une protéine de poids moléculaire proche de 23 kDa.

Méthode CPG (chromatographie en phase gazeuse) employée pour quantifier les 1,2-diacylglycérol

Le dosage est réalisé par chromatographie en phase gazeuse à l'aide d'un étalon interne, le 1,3-dipalmitine. L'analyse est réalisée sur une colonne capillaire de type apolaire (HP5 (30 m x 0.25 mm x 0.25 mm), a l'aide de Chromatographie en phase gazeuse Agilent 7890 pilotée par le logiciel ChemStation® et possédant un détecteur à ionisation de flamme et un injecteur automatique.

Le gaz vecteur étant de l'Hélium (1.0 ml/min), l'injecteur et le détecteur étant chauffés à 330°C, le four étant programmé en isotherme à 325°C pendant 30 minutes. Le volume d'échantillon injecté étant de 1μl. Dans ces conditions, le 1,3-dipalmitine a un temps de rétention respectif de 13 minutes. Les différents diacylglycérols possèdent un temps de rétention déterminé par l'utilisation de standards, des études bibliographiques préalables ainsi que des analyses préliminaires réalisées par Chromatographie en phase gazeuse couplée à de la spectrométrie de masse.

Détermination des coefficients de réponse, l'hypothèse de travail étant que tous les 1,2-diacylglycérols ont le même coefficient de réponse que la 1,2-dioléine, la 1,2-dioléine est utilisée comme molécule de référence.

Solutions étalons : préparer quatre solutions comme suit en pesant environ exactement les masses suivantes :

Tableau 5 - Préparation de solutions étalons

| Produit | Solution 1 | Solution 2 | Solution 3 | Solution 4 |
|---|---|---|---|---|
| **1,3-dipalmitine** | 20 mg | 20 mg | 10 mg | 10 mg |
| **1,2-dioléine** | 20 mg | 10 mg | 20 mg | 10 mg |
| **Pyridine** | 500 μL | | | |
| **HMDS** | 400 μL | | | |
| **TFA** | 50 μL | | | |

Dans nos conditions d'analyse, le coefficient de réponse du 1,2-dioléine est de 1,2315 (avec un CV de 1,63%).

L'huile enrichie est ensuite analysée comme suit :

Tableau 6 - Analyse de l'huile enrichie

| Produit | Solution 5 | Solution 6 |
|---|---|---|
| **Huile enrichie en 1,2-diacylglycérols** | 100 mg | 100 mg |
| **1,3-dipalmitine** | 15 mg | 15 mg |
| **Pyridine** | 500 μL | |
| **HMDS** | 400 μL | |
| **TFA** | 50 μL | |

La quantité de 1,2-diacylglycérol contenu dans la solution est ensuite déterminée comme suit :Calcul de la masse de 1,2-dioléine contenu dans la solution :

$$m(Ech) = [\alpha(Ech) \times A(Ech) \times m(SI)] / A(SI)$$

Avec : m = masse (en mg)
A = aire sous le pic
$\alpha$ = Coefficient de réponse
Ech = échantillon (1,2-diacylglycérols)
SI = standard interne (1,3-dipalmitine)

soit :

$$m(1{,}2\text{-diacylglycérols}) = [1{,}2315 \times \Sigma\ A(1{,}2\text{-diacylglycérols}) \times m(1{,}3\text{-dipalmitine})] / A(1{,}3\text{-dipalmitine})$$

Calcul de la teneur : Teneur (%) = [m(1,2-diacylglycérols) / m(huile enrichie)] x 100

**Exemple 1 - Procédé d'obtention de la fraction non hydrosoluble du suc de *Carica papaya,* enrichie en lipase de *Carica papaya***

L'extrait enrichi en lipase de *Carica papaya* est obtenu en réalisant une suspension de 100 g de suc de papaye brut séché dans 900 g d'eau distillé. Le suc de papaye brut séché se présente ainsi comme une suspension biphasique comprenant des particules en suspension et une fraction soluble dans l'eau.
Le mélange est agité à l'aide d'un agitateur de type ancre à une vitesse de 500 rotations par minutes (rpm) pendant 2 heures à température ambiante.
La fraction insoluble dans l'eau (non hydrosoluble) du suc de papaye (telle que caractérisée ci-dessus) est séparée de la fraction soluble par centrifugation du mélange à l'aide d'une centrifugeuse, type centrifugeuse à assiette à débit de 200 litres par heure, pendant 30 minutes.
Le culot de centrifugation présente la fraction d'intérêt, c'est-à-dire la fraction insoluble dans l'eau du suc de papaye brut.
On obtient ainsi 20g de fraction particulaire possédant un poids sec de 25%.

**Exemple 2 - Procédé de préparation d'une huile activée, enrichie en 1.2-diacylglycérol**

Le mélange est préparé en deux étapes distinctes, puis en réunissant, par simple mélange, les produits issus de ces deux procédés dans des proportions adéquates.
L'huile enrichie en 1,2-diacylglycérols est préparée à partir d'huile vierge de Calophylle Inophyle ou Framboise ou Baobab ou Onagre ou Noix du Brésil ou Camélia (en raison de leurs excellentes propriétés cosmétiques au niveau cutané lorsqu'elles sont activées, au sens de la présente invention) et d'une quantité d'eau ou de solution saline contenant un ion bivalent (tel qu'un ion calcium ou magnésium), représentant de 0,5 à 50%, et de préférence de 1 à 40%, du volume de l'huile. Dans une cuve thermostatée, ce mélange est maintenu à une température comprise entre 30 et 70°C, préférentiellement à une température de 50°C. Ce mélange est maintenu sous agitation à l'aide d'un équipement de

type rotor stator afin de permettre la formation d'une émulsion entre l'huile et l'eau.

Ce mélange est ensuite maintenu sous agitation en présence d'un volume donné de la fraction non hydrosoluble du suc de Carica papaya, enrichie en lipase de Carica papaya, obtenue en mettant en œuvre le procédé de l'exemple 1, dans un rapport en volume fraction non hydrosoluble du suc de Carica papaya/corps gras utilisé compris entre environ 0,01 et environ 0,2, avantageusement entre environ 0,05 et 0,015, de préférence d'environ 0,1.

L'agitation et la température sont maintenues pendant une durée comprise entre une et six heures (préférentiellement quatre heures) c'est-à-dire pendant un temps suffisant pour que la quantité de diacylglycérols formée soit maximale.

Le suivi de la réaction est effectué par la détermination d'indicateurs classiques dans l'industrie des corps gras (mesure de l'indice d'acide NF EN ISO 660 et de peroxyde NF ISO 3976), et par l'utilisation de méthodes chromatographiques, notamment par chromatographie en phase gazeuse permettant de qualifier et de quantifier la teneur en diacylglycérols.

Le milieu réactionnel est ensuite purifié par des techniques de purification physico-chimiques, le but étant d'obtenir une huile enrichie en 1,2-diacylglycérols, brillante, inodore et possédant une couleur la plus proche de l'huile initialement utilisée pour la réaction.

Le milieu réactionnel est dans un premier temps filtré sur une série de filtres de cellulose de porosité décroissante (500 à 250$\mu$m), ceci permettant dans un premier temps la rupture de l'émulsion et dans un deuxième temps la clarification du milieu.

L'eau résiduelle est ensuite éliminée à l'aide d'un agent dessiccateur de type sulfate de magnésium anhydre. L'huile ainsi obtenue est ensuite désodorisée et rendue brillante à l'aide d'un charbon actif (préférentiellement du charbon actif Cabot Norit® type CN1).

Les caractéristiques de l'huile ainsi obtenue possède une partie commune inhérente au procédé décrit dans l'invention mais aussi des caractéristiques spécifiques intrinsèques à la nature de l'huile utilisée. Le milieu réactionnel est donc constitué, principalement de triglycérides non transformés, de monoglycérides et de diacylglycérols de type 1,2 et 1,3.

Les huiles sont communément caractérisées par un indice d'acide compris entre 15 et 50, un indice de péroxyde compris entre 5 et 30 et un taux de 1.2-diacylglycérol compris entre 5 et 30%.

La nature des diacylglycérols obtenus est fonction des triacylglycérols initialement présents dans l'huile.

Les tableaux 7 et 8 infra permettent de comparer l'indice d'acide, l'indice de peroxyde et la teneur en 1,2-diacylglycérols et en 1,3-diacylglycérol de :

- six huiles vierges, particulièrement préférées au sens de la présente invention, avec
- les mêmes six huiles vierges enrichies en 1,2-diacylglycérols.

Tableau 7 - indice d'acide, indice de peroxyde et teneur en 1,2-diacvlqlvcérols et en 1,3 diacylglycérol des six huiles vierges préférées

| **Huile vierge initiale** | **Huile de Framboise** | **Huile de Baobab** | **Huile d'Onagre** | **Huile de noix du Brésil** | **Huile de Camélia** | **Huile de Calophylle Inophylle** |
|---|---|---|---|---|---|---|
| **Indice d'acide (mg/g)** | 4 | 5 | 4 | 3 | 5 | 20 |
| **Indice de Peroxyde (meq $0_2$ / Kg)** | 15 | 14 | 10 | 14 | 12 | 8 |
| **Teneur en 1,2-diacylglyc érols (%)** | 0.5 | 0 | 0 | 0 | 0.3 | 2 |
| **Teneur en 1,3-diacylglyc érol** | 0.5 | 0 | 0 | 0 | 0.4 | 1 |

Tableau 8 - indice d'acide, indice de peroxyde et teneur en 1,2-diacylglycérols et en 1,3-diacylglycérol des six huiles vierges préférées, après enrichissement en 1,2 DAG

| Huile après enrichissement | Huile de Framboise enrichie en 1,2-Dag | Huile de Baobab enrichie en 1,2-Dag | Huile d'Onagre enrichie en 1,2-Dag | Huile de noix du Brésil enrichie en 1,2-Dag | Huile de Camélia enrichie en 1,2-Dag | Huile de Calophylle Inophylle enrichie en 1,2-Dag |
|---|---|---|---|---|---|---|
| **Indice d'acide (mg/g)** | 21,8 | 27,1 | 21,6 | 18,2 | 22,3 | 48,6 |
| **Indice de Péroxyde (meq $0_2$ / Kg)** | 29,6 | 2,5 | 25,8 | 18,7 | 12,9 | 4,2 |
| **Teneur en 1,2-diacylglycérois (%)** | 12 | 16 | 16.9 | 17.7 | 18 | 8 |
| **Teneur en 1,3-diacylglycérol** | 2 | 3.5 | 2.5 | 2 | 2 | 1.5 |

Par ailleurs, une cire liquide enrichie en alcool gras est préparée en mettant en œuvre *mutatis mutandis* le procédé de préparation décrit supra, à savoir en utilisant une cire à la place d'une huile et en obtenant une cire enrichie en alcools gras. Dans un souci d'exhaustivité, ce procédé est décrit ci-après :

Dans un premier temps de la cire de jojoba est émulsionnée à l'aide d'un agitateur de type rotor stator, en mélangeant ladite cire avec une quantité d'eau ou de solution saline contenant un ion bivalent tel qu'un ion calcium ou magnésium, représentant de 0,5 à 50%, et de préférence de 1 à 40%, du volume de cire, sans modification de pH. Dans une cuve thermostatée, ce mélange est maintenu à une température comprise entre 30 et 70°C, préférentiellement à une température de 50°C. Ce mélange est maintenu sous agitation à l'aide d'un équipement de type rotor stator afin de permettre la formation d'une émulsion stable entre la cire et l'eau.

Ce mélange est ensuite maintenu sous agitation en présence d'un volume donné de la fraction non hydrosoluble du suc de *Carica papaya,* enrichie en lipase de *Carica papaya,* obtenue en mettant en œuvre le procédé de l'exemple 1, dans un rapport en volume fraction non hydrosoluble du suc de *Carica papaya*/cire utilisée compris entre environ 0,01 et environ 0,2, avantageusement entre environ 0,05 et 0,015, de préférence d'environ 0,1.

L'agitation et la température sont maintenues pendant une durée comprise entre une et six heures (préférentiellement six heures) c'est-à-dire pendant un temps suffisant pour que la quantité d'alcools gras formée soit maximale.

Le milieu réactionnel est dans un premier temps filtré sur une série de filtres de cellulose de porosité décroissante (500 à 250$\mu$m), ceci permettant dans un premier temps la rupture de l'émulsion et dans un deuxième temps la clarification du milieu.

L'eau résiduelle est ensuite éliminée à l'aide d'un agent dessiccateur de type sulfate de magnésium anhydre. La cire ainsi obtenue est ensuite désodorisée et rendue brillante à l'aide d'un charbon actif (préférentiellement du charbon actif Cabot Norit® type CN1).

Le produit de réaction est effectué par la mesure de l'indice d'acide NF EN ISO 660 et par l'utilisation de méthodes chromatographiques, notamment par chromatographie en phase gazeuse permettant de qualifier et de quantifier la teneur en acides gras et alcools gras.

Le produit obtenu est, de préférence, caractérisé par une teneur en alcools gras comprise entre 2 et 4 %, un indice d'acide de 5 mg/g de cire.

Dans un mode de réalisation particulier de l'invention, la composition correspond à une huile enrichie en 1,2-diacylglycérols (huile activée) ou un mélange de plusieurs huiles enrichies en 1,2-diacylglycérols (huiles activées).

Dans un mode de réalisation particulier de l'invention, la composition correspond à une cire liquide enrichie en alcool gras (cire activée) ou un mélange de plusieurs cires liquides enrichies en alcool gras (cires activées).

Dans un mode de réalisation préférée, la composition selon l'invention est ensuite obtenue en mélangeant simplement l'huile enrichie en 1,2-diacylglycérols (huile activée) et la cire liquide enrichie en alcool gras (cire activée) dans des proportions pondérales comprises entre 100 et 90% d'huile enrichie et 0 à 10% de cire liquide enrichie.

**Exemple 3 Formulations cosmétiques incluant une huile activée**

3.1-Gel contour des yeux

| INGREDIENTS | NOM COMMERCIAL / NOM INCI | % p/p |
|---|---|---|
| Phase A | | |
| Purified Water | Water/Aqua | Qs 100 |
| **Mélange huile de tamanu activée/cire de jojoba activée selon exemple 2** | (Proposed) Hydrolyzed Calophyllum Inophyllum Seed Oil and Hydrolyzed Jojoba Esters | 1.50 |
| **Flexithix ™ polymer** | PVP | 3.00 |
| Phase B | | |
| **Si-Tec™ DM 350 silicones** | Dimethicone | 3.00 |
| **Si-Tec™ RE-100 silicones** | Cyclopentasiloxane (and) Dimethicone/Vinyltrimethylsiloxysilicate Crosspolymer | 10.00 |
| Cyclopentasiloxane | Cyclopentasiloxane | 5.00 |
| Phase C | | |
| **Liquid germall plus preservative** | Propylene glycol (and) Diazolidinyl urea (and) Iodopropynyl butylcarbamate | 0.50 |
| BPD-500 | HDI/Trimethylol hexyllactone crosspolymer & Silica | 0.50 |
| Total | | 100.00 |

Tableau 9 – composition d'un gel « contour des yeux » selon l'invention

Propriétés :

| | |
|---|---|
| Apparence: | Gel lisse, semi-transparent |
| pH: | 5,50 - 6,0 |
| Viscosité (D0) | 15000 - 25000 (Brookfield RVT/Spindle B/5 RPM/ 1 minute/25°C) |

Cette formule a subi un test de stabilité accélérée de 3 mois en laboratoire. La conservation de cette formule a été validée par un double test d'efficacité sur 28 jours. Toutefois, les conservateurs n'ont pas été optimisés à leur plus bas niveau d'efficacité.

3.2-Sérum visage biphasique

| INGREDIENTS | NOM COMMERIAL / NOM INCI | % p/p |
|---|---|---|
| Phase A | | |
| Purified Water | Water/Aqua | Qs 100 |
| Phase B | | |
| **Blanose™ 7H3SF CMC** | **Carboxymethylcellulose** | **0.30** |
| Zemea* | Propanediol | 1.00 |
| Phase C | | |
| **Rokonsal™/Liquapar™ MEP preservative** | **Phenoxyethanol (and) Methylparaben (and) Ethylparaben (and) Propylparaben** | **0.70** |
| **Neomatrix™ biofunctional** | **Water/Aqua (and) Glycerin (and) Pentapeptide (proposed name)** | **1.00** |
| Sodium Chloride | Sodium Chloride | 1.00 |
| Unicert* Blue 05601-J (sol. 0.1%) | Water/Aqua (and) CI 42090 (FD&C Blue No.1) | 0.20 |

| | | |
|---|---|---|
| Unicert* Yellow 08005-J (sol 0.1%) | Water/Aqua (and) CI 19140 (FD&C Yellow No.5) | 0.60 |
| Phase D | | |
| **Ceraphyl™ ODS ester** | **Octyldodecyl Stearate** | **7.00** |
| Unicert* Green K7016-J | CI 61565 (D&C Green No.6) | 0.006 |
| Phase E | | |
| **Optiphen™ preservative** | **Phenoxyethanol (and) Caprylyl Glycol** | **0.50** |
| **Ceraphyl™ 375 ester** | **Isostearyl Neopentanoate** | **3.00** |
| **Mélange huile de tamanu activée/cire de jojoba activée selon exemple 2** | (Proposed) Hydrolyzed Calophyllum Inophyllum Seed Oil and Hydrolyzed Jojoba Esters | 2.50 |
| Smart* 5 | Isododecane (and) Hydrogenated tetradecenyl/methylpentadecene | 8.00 |
| DC* FZ-3196 | Caprylyl Methicone | 5.00 |
| PF Absolute Perfection | Parfum/Fragrance (and) Linalool | 0.10 |
| Total | | 100.00 |

Tableau 10-Composition d'un sérum visage biphasique selon l'invention

Propriétés :

| | |
|---|---|
| Apparence: | Liquide biphasique vert et bleu foncé - agiter avant usage |
| pH: | 5,0 - 6,0 |
| Viscosité (D0) | N.A. |

Cette formule a subi un test de stabilité accélérée de 3 mois en laboratoire. La conservation de cette formule a été validée par un double test d'efficacité sur 28 jours. Toutefois, les conservateurs n'ont pas été optimisés à leur plus bas niveau d'efficacité.

**Exemple 4 - Etude de l'expression des cytokératines dans la peau humaine *ex vivo,* en présence de différentes huiles activées selon l'invention**

4.1 - Introduction sur les cytokératines

Les cytokératines sont des protéines des filaments intermédiaires, qui conjointement avec d'autres protéines, forment le cytosquelette des cellules. Elles ont de nombreuses fonctions comprenant le maintien de la structure épithéliale, la protection contre les blessures et la communication avec d'autres composants cytoplasmiques (*Fuertes L. et al., 2013*).

Elles sont exprimées par paires, avec des profils d'expression différents suivant leur localisation et elles sont classées numériquement de 1 à 20 selon leur poids moléculaire et point isoélectrique. Les cytokératines sont classées en deux groupes (*Fuertes L. et al., 2013*):

- les cytokératines de type I, qui sont acides et correspondent généralement à des cytokératines de bas poids moléculaires,
- les cytokératines de type II, qui sont basiques et ont généralement un haut poids moléculaire.

Lors du processus de différenciation épidermique, les kératinocytes de la couche basale perdent leur potentiel prolifératif, migrent vers les couches supérieures et l'expression des kératines 5 et 14 est interrompue tandis que celle des kératines 1 et 10 augmente (*Paladini R.D. et al., 1999*).

4.2 - But de l'étude

Le but de cette étude est de déterminer l'influence de différents mélanges huiles activées/cire activée selon l'invention, obtenus en mettant en œuvre le procédé de l'exemple 2, sur l'expression des cytokératines dans la peau humaine *ex vivo.*

4.3 - Huiles testées

Les différentes huiles testées sont :

Mélange huile de tamanu (calophylle Inophylle) activée/cire de jojoba activée (abrégé dans le reste de l'exemple 4 en « huile de tamanu activée ») obtenu par le procédé de l'exemple 2, versus huile de tamanu vierge native,

Mélange huile de baobab activée/ cire de jojoba activée (abrégé dans le reste de l'exemple 4 en « huile de baobab activée ») obtenu par le procédé de l'exemple 2 versus huile de baobab vierge native,

Mélange huile de framboise activée/ cire de jojoba activée (abrégé dans le reste de l'exemple 4 en « huile de framboise activée ») obtenu par le procédé de l'exemple 2 versus huile de framboise vierge native,

Mélange huile d'onagre activée/ cire de jojoba activée (abrégé dans le reste de l'exemple 4 en « huile d'onagre activée ») obtenu par le procédé de l'exemple 2 versus huile d'onagre vierge native.

4.3 - Protocole

Des échantillons de peau humaine de 6 mm de diamètre sont mis en culture à l'interface air/liquide. Les huiles activées sont diluées à 0,5% dans l'huile non activée correspondante. 20 $\mu$L d'huile sont appliqués topiquement sur les échantillons de peau (une seule application), puis ces biopsies sont incubées durant 24 heures à 37°C. A la fin de la culture, les échantillons de peau humaine sont inclus en gel OCT (*optimal cutting temperature*). L'OCT prend en masse lors du contact avec le froid (azote liquide). Ces blocs contenant les échantillons de peau sont conservés à -20°C.

Des coupes de 6 $\mu$m d'épaisseur sont ensuite réalisées. Ces coupes d'échantillons de peau sont ensuite séchées à l'étuve à 37°C, puis fixées avec de l'acétone (refroidi au préalable à -20°C).

L'immunomarquage est réalisé à l'aide d'un anticorps monoclonal de souris spécifique de pancytokératine (Abcam, Réf. ab27988), puis d'un anticorps secondaire anti-souris couplé à un fluorochrome (Invitrogen, Réf. A21202), puis les coupes d'échantillons de peau sont alors examinées au microscope à Epi-fluorescence (Zeiss Axiovert 200M). Une quantification de la fluorescence, à partir des photographies obtenues, a été réalisée avec le logiciel Volocity.

4.4 - Résultats

Les observations microscopiques montrent une fluorescence significativement plus intense au niveau de l'épiderme des biopsies traitées avec l'huile de tamanu activée, l'huile de baobab activée, l'huile de framboise activée et l'huile d'onagre activée par rapport à l'huile non activée correspondante. Les données obtenues sont présentées ci-après, au sein des tableaux 11-14.

Tableau 11 - Expression des cytokératines (%) huile de tamanu activée vs. huile de tamanu vierge

| | 0,5% Huile de tamanu activée | Huile de tamanu vierge |
|---|---|---|
| Expression des cytokératines (%) | 123,3 ± 4,6 | 100 ± 4,7 |

Tableau 12 - Expression des cytokératines (%) huile de baobab activée vs. huile de baobab vierge

| | 0,5% Huile de baobab activée | Huile de baobab vierge |
|---|---|---|
| Expression des cytokératines (%) | 112,7 ± 2,6 | 100 ± 3,1 |

Tableau 13 - Expression des cytokératines (%) huile de framboise activée vs. huile de framboise vierge

| | 0,5% Huile de framboise activée | Huile de framboise vierge |
|---|---|---|
| Expression des cytokératines (%) | 124,7 ± 2,8 | 100 ± 2,2 |

Tableau 14 - Expression des cytokératines (%) huile d'onagre activée vs. huile d'onagre vierge

| | 0,5% Huile d'onagre activée | Huile d'onagre vierge |
|---|---|---|
| Expression des cytokératines (%) | 124,8 ± 4,6 | 100 ± 4,2 |

L'analyse statistique a été réalisée *versus* l'huile non activée correspondante (Moyenne ± sem ; n=8 ; *** : hautement significatif avec le test *t* de Student).

Afin de mettre en exergue la différence d'expression observée entre les huiles activées et les huiles vierges correspondantes, les données obtenues ont été reportées sur le graphique montré en figure 5.

4.5 - Conclusions

L'expression des cytokératines est augmentée dans la peau humaine *ex vivo* par l'huile de tamanu activée, l'huile de baobab activée, l'huile de framboise activée et l'huile d'onagre activée. Ainsi, ces huiles activées stimulent la différenciation épidermique et possèdent donc des propriétés cosmétiques intéressantes.

4.6 - Références bibliographiques

Fuertes L., Santonja C., Kutzner H. and Requena L. Immunohistochemistry in Dermatopathology : a review of the most commonly used antibodies (Part I). Actas Dermo-Sifiliográficas. 104(2):99-127 (2013)

Paladini R.D. and Coulombe P.A. The functional diversity of epidermal keratins revealed by the partial rescue of the keratin 14 null phenotype by keratin 16. The Journal of Cell Biology. 146 (5):1185-1201 (1999)

## Revendications

**1.** Procédé d'obtention de la fraction non hydrosoluble du suc de *Carica papaya,* enrichie en lipase de *Carica papaya,* ledit procédé comprenant les étapes suivantes :

a) mettre en suspension du suc de *Carica papaya* brut séché dans de l'eau distillée, dans un rapport en poids *Carica papaya* brut séché/eau distillée compris entre 0,01 et 0,5, de préférence entre 0,05 et 0,25, préférablement entre 0,08 et 0,2 ; et de manière particulièrement préférée d' 0,1,
b) centrifuger la suspension obtenue à l'étape a) de manière à obtenir un culot contenant ladite fraction non hydrosoluble du suc de *Carica papaya,* durant un intervalle de temps compris entre 5 minutes et 90 minutes, préférablement compris entre 15 minutes et 60 minutes, de manière préférée compris entre 20 et 40 minutes, et à une vitesse de rotation comprise entre 2000 et 6000 rpm, préférablement comprise entre 3000 et 5000 rpm, de manière préférée de 4000 rpm.
c) récupérer le culot contenant ladite fraction non hydrosoluble du suc de *Carica papaya,* enrichie en lipase de *Carica papaya,*

ledit procédé comprenant, après l'étape a) et avant l'étape b), l'étape suivante :

a') agiter la suspension obtenue à l'étape a) durant un intervalle de temps compris entre 15 minutes et 240 minutes, avantageusement durant un intervalle de temps compris entre 30 minutes et 180 minutes, préférablement entre 60 minutes et 150 minutes, et de manière particulièrement préférée de 120 minutes, et à une température comprise entre 10°C et 30°C, avantageusement à une température comprise entre 15°C et 25°C, de préférence à température ambiante,
ledit procédé comprenant, après l'étape b) et avant l'étape c), l'étape suivante :
b') sécher le culot humide jusqu'à obtention d'une poudre particulaire ; la fraction non hydrosoluble de *Carica papaya,* enrichie en lipase de *Carica papaya,* étant récupérée, à l'étape c), sous forme de poudre particulaire.

**2.** Utilisation de la fraction non hydrosoluble de *Carica papaya* obtenue selon le procédé de la revendication 1 pour enrichir un corps gras en diacylglycérols et/ou une cire en alcools gras.

**3.** Procédé de préparation d'un corps gras enrichi en diacylglycérols, ledit procédé comprenant les étapes suivantes :

i) mélanger un corps gras avec de l'eau ou avec une solution saline contenant de préférence un ion bivalent, tel qu'un ion calcium ou magnésium, dans un rapport en volume eau ou solution saline/corps gras compris entre 0,005 et 0,5, préférablement entre 0,01 et 0,4,
ii) maintenir ce mélange sous agitation jusqu'à obtention d'une émulsion entre le corps gras et l'eau, à une température comprise entre 30°C et 70°C, avantageusement d' 50°C, de préférence durant un intervalle de temps compris entre 1 et 30 minutes, préférablement entre 5 et 20 minutes, et de préférence de 10 minutes,
iii) toujours sous agitation et à ladite température, mettre en présence le mélange obtenu à l'étape précédente avec un volume donné de la fraction non hydrosoluble du suc de *Carica papaya* obtenu selon le procédé de la revendication 1, dans un rapport en volume fraction non hydrosoluble du suc de *Carica* papaya/corps gras utilisé compris entre 0,01 et 0,2, avantageusement entre 0,05 et 0,015, de préférence d' 0,1,
iv) maintenir ladite température et l'agitation durant un intervalle de temps compris entre une heure et six heures, avantageusement entre deux heures et cinq heures, de préférence durant quatre heures, afin d'obtenir le corps gras enrichi en diacylglycérols.

**4.** Procédé selon la revendication précédente comprenant, après l'étape iv), l'étape suivante :
v) filtrer le mélange obtenu à l'étape iv), de préférence en utilisant au moins un filtre à gradient ou au moins une superposition d'au moins deux filtres de porosité décroissante, de 500 $\mu$m à 250 $\mu$m, de façon à séparer ladite fraction non hydrosoluble du suc de *Carica papaya,* enrichie en lipase de *Carica papaya,* du milieu réactionnel contenant le corps gras enrichi en diacylglycérols.

**5.** Procédé selon la revendication 4, ledit procédé comprenant, après l'étape v), au moins l'une des deux étapes suivantes, et avantageusement les deux :

- éliminer l'eau résiduelle présente dans le milieu réactionnel contenant le corps gras enrichi en diacylglycérols au moyen d'un agent dessiccateur tel que du sulfate de magnésium anhydre, et/ou
- désodoriser et/ou améliorer la brillance du corps gras enrichi en diacylglycérols au moyen d'un charbon actif.

**6.** Procédé selon l'une des revendications 3 à 5, dans lequel ledit corps gras est une huile d'origine animale, végétale et/ou marine, vierge ou raffinée, avantageusement vierge, ladite huile étant préférablement d'origine végétale et/ou marine, de préférence d'origine végétale.

**7.** Procédé selon l'une des revendications 3 à 6, dans lequel ledit corps gras est une huile comprenant des triglycérides possédant :

- des chaînes hydrocarbonées aliphatiques d'un acide gras possédant un nombre de carbone compris entre 12 et 26, avantageusement entre 16 et 20, et de manière particulièrement préférée entre 16 et 18 ; ladite chaîne hydrocarbonée aliphatique étant linéaire ou ramifiée, avantageusement linéaire, saturée ou insaturée, avantageusement insaturée, préférablement avec un nombre d'insaturations compris entre 1 et 6, et de préférence compris entre 1 et 3 ; ou
- des chaînes mono- ou poly- hydroxylées, et/ou mono- ou poly- méthoxylées, et/ou mono- ou poly- oxydées, et/ou mono- ou poly-époxylées.

**8.** Procédé selon l'une des revendications 3 à 7, dans lequel ledit corps gras est une huile, de préférence vierge, sélectionnée parmi l'huile de calophylle inophyle, l'huile de framboise, l'huile de camélia, l'huile d'onagre, l'huile de noix du Brésil, l'huile de baobab et l'huile d'olive, ou un mélange d'au moins deux de ces huiles ; ladite huile, de préférence vierge, étant avantageusement sélectionnée parmi l'huile de calophylle inophyle, l'huile de framboise, l'huile de camélia, l'huile d'onagre, l'huile de noix du Brésil et l'huile de baobab, ou un mélange d'au moins deux de ces huiles ; de manière particulièrement préféré ledit corps gras étant l'huile de calophylle inophyle, de préférence l'huile de calophylle inophyle vierge.

**9.** Procédé selon l'une des revendications 3 à 8, dans lequel lesdits diacylglycérols consistent en des 1,2-diacylglycérols et 1,3-diacylglycérols respectivement représentés par les formules (I) et (II) suivantes :

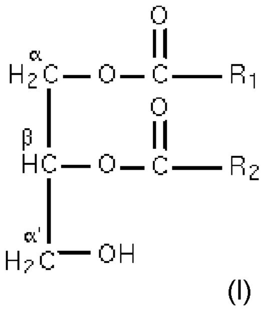
(I)
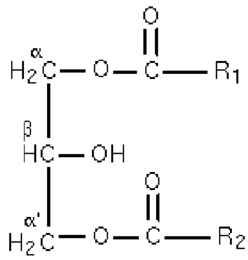
(II)

dans lesquelles R1 et R2 sont :

- des chaînes hydrocarbonées aliphatiques d'un acide gras possédant un nombre de carbone est compris entre 12 et 26, avantageusement entre 16 et 20, et de manière particulièrement préférée entre 16 et 18 ; ladite chaîne hydrocarbonée aliphatique étant linéaire ou ramifiée, avantageusement linéaire, saturée ou insaturée, avantageusement insaturée, préférablement avec un nombre d'insaturations compris entre 1 et 6, et de préférence compris entre 1 et 3 ; ou
- des chaînes mono- ou poly- hydroxylées, et/ou mono- ou poly- méthoxylées, et/ou mono- ou poly- oxydées, et/ou mono- ou poly-epoxylées.

**10.** Procédé selon l'une des revendications 3 à 9, dans lequel lesdits diacylglycérols comprennent majoritairement, et de préférence consistent essentiellement en, des 1,2-diacylglycérols représentés par la formule (I) suivante :

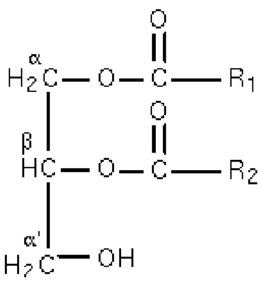
(I)

dans laquelle R1 et R2 sont :

- des chaînes hydrocarbonées aliphatiques d'un acide gras possédant un nombre de carbone est compris entre 12 et 26, avantageusement entre 16 et 20, et de manière particulièrement préférée entre 16 et 18 ; ladite chaîne hydrocarbonée aliphatique étant linéaire ou ramifiée, avantageusement linéaire, saturée ou insaturée, avantageusement insaturée, préférablement avec un nombre d'insaturations compris entre 1 et 6, et de préférence compris entre 1 et 3 ; ou
- des chaînes mono- ou poly- hydroxylées, et/ou mono- ou poly- méthoxylées, et/ou mono- ou poly- oxydées, et/ou mono- ou poly-epoxylées.

**11.** Procédé de préparation d'une cire enrichie en alcools gras, mettant en œuvre le procédé selon l'une des revendications 3 à 5, dans lequel l'on utilise, à titre de corps gras, une cire et l'on obtient, en lieu et place d'un corps gras enrichi en diacylglycérols, une cire enrichie en alcools gras, avantageusement en alcools gras de formule (III) :

(III) R" - OH

dans laquelle R" représente :

- la chaîne hydrocarbonée aliphatique d'un alcool gras comportant de 10 à 34 atomes de carbone, de préférence de 12 à 26 atomes de carbone, et de manière particulièrement préférée de 16 à 22 atomes de carbone ;
- ladite chaîne hydrocarbonée aliphatique étant linéaire ou ramifiée, avantageusement linéaire, saturée ou insaturée, préférablement avec maximum de 6 insaturations ; ou
- une chaîne mono- ou poly- hydroxylée, et/ou mono- ou poly-méthoxylée, et/ou mono- ou poly-oxydée, et/ou

mono- ou poly-époxylée.

12. Procédé selon la revendication 11, dans lequel ladite cire est sélectionnée parmi :

- une cire d'origine animale, comme par exemple la cire d'abeille,
- une cire d'origine minérale, ou, de préférence,
- une cire d'origine végétale, comme la cire de jojoba, da carnauba, de candelilla,
- ou leur(s) mélange(s) ; ladite cire étant avantageusement la cire de jojoba.

13. Corps gras enrichi en diacylglycérols, obtenu par le procédé selon l'une des revendications 3 à 10, dans lequel ledit corps gras est une huile vierge sélectionnée parmi l'huile de calophylle inophyle, l'huile de framboise, l'huile de camélia, l'huile d'onagre, l'huile de noix du Brésil, l'huile de baobab ou un mélange d'au moins deux de ces huiles ; ladite huile, étant avantageusement sélectionnée parmi l'huile de calophylle inophyle, l'huile de framboise, l'huile de camélia, l'huile d'onagre, l'huile de noix du Brésil et l'huile de baobab, ou un mélange d'au moins deux de ces huiles ; de manière particulièrement préféré ledit corps gras étant l'huile de calophylle inophyle.

14. Corps gras enrichi en diacylglycérols selon la revendication 13, ledit corps gras ayant une teneur en diacylglycérols comprise entre 5% et 30%, de préférence entre 7% et 18%, un indice d'acide compris entre 15 et 50 mg/g, et un indice de peroxyde compris entre 5 et 30 mg/g, de préférence inférieur à 20 mg/g.

15. Composition comprenant un mélange d'au moins un corps gras enrichi en diacylglycérols selon l'une des revendications 13 ou 14 et d'au moins une cire enrichie en alcools gras obtenu selon le procédé de l'une des revendications 11 ou 12, dans un rapport en poids cire enrichie en alcools gras/corps gras enrichi en diacylglycérols compris entre 0,01 et 0,11, préférablement entre 0,02 et 0,08, et de préférence de 0,03.

16. Utilisation cosmétique d'une quantité efficace d'au moins un corps gras enrichi en diacylglycérols selon l'une des revendications 13 ou 14, et d'au moins une cire enrichie en alcools gras obtenu selon le procédé d'une des revendications 11 ou 12, ou d'une composition selon la revendication 15, par application cutanée, pour retarder l'apparition ou limiter les signes du vieillissement cutané choisis parmi les rides et ridules, les défauts pigmentaires tels que les taches de vieillesse ou le manque d'éclat, les poches sous les yeux, les cernes, le flétrissement, la perte d'élasticité, de fermeté et/ou de tonus de la peau, l'amincissement de la peau ou encore toute dégradation interne de la peau consécutive à un stress environnemental tel que la pollution, chez un individu humain ou animal sain, de préférence humain

17. Composition topique comprenant une quantité efficace d'au moins un corps gras enrichi en diacylglycérols selon l'une des revendications 13 ou 14, et d'au moins une cire enrichie en alcools gras obtenu selon le procédé d'une des revendications 11 ou 12, ou d'une composition selon la revendication 15, pour son utilisation pour protéger la peau et/ou les phanères contre les agressions extérieures dues aux rayonnement UVA et UVB, chez un individu humain ou animal sain.

## Patentansprüche

1. Verfahren zum Erhalten der wasserunlöslichen Fraktion des Saftes von *Carica papaya,* angereichert mit *Carica-papaya*-Lipase, wobei das Verfahren die folgenden Schritte umfasst:

a) Suspendieren des Safts von roher getrockneter *Carica papaya* in destilliertem Wasser in einem Gewichtsverhältnis von getrockneter roher *Carica papaya* zu destilliertem Wasser zwischen 0,01 und 0,5, vorzugsweise zwischen 0,05 und 0,25, bevorzugt zwischen 0,08 und 0,2; und besonders bevorzugt von 0,1,
b) Zentrifugieren der in Schritt a) erhaltenen Suspension, um ein Pellet zu erhalten, das die wasserunlösliche Fraktion des Saftes von *Carica papaya* enthält, während eines Zeitraums zwischen 5 Minuten und 90 Minuten, vorzugsweise zwischen 15 Minuten und 60 Minuten, bevorzugt zwischen 20 und 40 Minuten, und bei einer Rotationsgeschwindigkeit zwischen 2.000 und 6.000 U/min, vorzugsweise zwischen 3.000 und 5.000 U/min, bevorzugt bei 4.000 U/min.
c) Gewinnen des Pellets, das die wasserunlösliche Fraktion des Saftes von *Carica Papaya,* angereichert mit *Carica-papaya*-Lipase, enthält,

wobei das Verfahren nach Schritt a) und vor Schritt b) den folgenden Schritt umfasst:

a') Rühren der in Schritt a) erhaltenen Suspension während eines Zeitraums zwischen 15 Minuten und 240 Minuten, vorteilhafterweise während eines Zeitintervalls zwischen 30 Minuten und 180 Minuten, vorzugsweise zwischen 60 Minuten und 150 Minuten, und besonders bevorzugt für 120 Minuten, und bei einer Temperatur zwischen 10 °C und 30 °C, vorteilhafterweise bei einer Temperatur zwischen 15 °C und 25 °C, vorzugsweise bei Raumtemperatur,
wobei das Verfahren nach Schritt b) und vor Schritt c) den folgenden Schritt umfasst:
b') Trocknen des Nasspellets, bis ein teilchenförmiges Pulver erhalten wird; wobei die wasserunlösliche Fraktion von *Carica papaya,* angereichert mit *Carica-papaya-Lipase,* in Schritt c) als teilchenförmiges Pulver gewonnen wird.

**2.** Verwendung der gemäß dem Verfahren nach Anspruch 1 erhaltenen wasserunlöslichen Fraktion von *Carica papaya,* um einen Fettstoff mit Diacylglycerinen und/oder ein Wachs mit Fettalkoholen anzureichern.

**3.** Verfahren zur Herstellung eines mit Diacylglycerin angereicherten Fettstoffs, wobei das Verfahren die folgenden Schritte umfasst:

i) Mischen eines Fettstoffs mit Wasser oder mit einer Salzlösung, die vorzugsweise ein zweiwertiges Ion enthält, wie z.B. Calcium- oder Magnesiumionen, in einem Volumenverhältnis von Wasser oder Salzlösung/Fettstoff zwischen 0,005 und 0,5, vorzugsweise zwischen 0,01 und 0,4,
ii) Halten dieser Mischung unter Rühren, bis sich eine Emulsion zwischen dem Fettstoff und dem Wasser ergibt, bei einer Temperatur zwischen 30 °C und 70 °C, vorzugsweise 50 °C, vorzugsweise für einen Zeitraum zwischen 1 und 30 Minuten, bevorzugt zwischen 5 und 20 Minuten, vorzugsweise 10 Minuten,
iii) stets unter Rühren und bei der genannten Temperatur Inkontaktbringen der in dem vorhergehenden Schritt erhaltene Mischung mit einem bestimmten Volumen der wasserunlöslichen Fraktion des gemäß dem Verfahren nach Anspruch 1 erhaltenen Saftes von *Carica papaya* in einem Volumenverhältnis der wasserunlöslichen Fraktion des Saftes von *Carica papaya zum* verwendeten Fettstoff zwischen 0,01 und 0,2, vorteilhafterweise zwischen 0,05 und 0,015, vorzugsweise 0,1,
iv) Halten der genannten Temperatur und Rühren über einen Zeitraum zwischen einer Stunde und sechs Stunden, vorteilhafterweise zwischen zwei Stunden und fünf Stunden, vorzugsweise über vier Stunden, um den mit Diacylglycerinen angereicherte Fettstoff zu erhalten.

**4.** Verfahren nach dem vorhergehenden Anspruch, umfassend nach Schritt iv) den folgenden Schritt:
v) Filtrieren der in Schritt iv) erhaltenen Mischung, vorzugsweise unter Verwendung mindestens eines Gradientenfilters oder mindestens einer Kombination von mindestens zwei Filtern mit abnehmender Porosität von 500 $\mu$m bis 250 $\mu$m, um die wasserunlösliche Fraktion des Saftes von *Carica papaya,* angereichert mit *Carica-papaya-Lipase,* von dem Reaktionsmedium abzutrennen, das den mit Diacylglycerinen angereicherten Fettstoff enthält.

**5.** Verfahren nach Anspruch 4, wobei das Verfahren nach Schritt v) mindestens einen der folgenden Schritte, vorzugsweise beide, umfasst:

- Entfernen des in dem Reaktionsmedium, das den mit Diacylglycerinen angereicherten Fettstoff enthält, vorhandenen Restwassers mit Hilfe eines Trocknungsmittels wie wasserfreiem Magnesiumsulfat, und/oder
- Desodorieren und/oder Verbessern des Glanzes des mit Diacylglycerinen angereicherten Fettstoffs mittels Aktivkohle.

**6.** Verfahren nach einem der Ansprüche 3 bis 5, wobei der Fettstoff ein Öl tierischen, pflanzlichen und/oder marinen Ursprungs, nativ oder raffiniert, vorteilhafterweise nativ, ist,
wobei das Öl vorzugsweise pflanzlichen und/oder marinen Ursprungs, bevorzugt pflanzlichen Ursprungs ist.

**7.** Verfahren nach einem der Ansprüche 3 bis 6, wobei der Fettstoff ein Öl ist, das Triglyceride umfasst, die Folgendes aufweisen:

- aliphatische Kohlenwasserstoffketten einer Fettsäure mit einer Kohlenstoffzahl zwischen 12 und 26, vorteilhafterweise zwischen 16 und 20, und besonders bevorzugt zwischen 16 und 18; wobei die aliphatische Kohlenwasserstoffkette linear oder verzweigt, vorteilhafterweise linear, gesättigt oder ungesättigt, vorteilhafterweise ungesättigt, vorzugsweise mit einer Anzahl von Ungesättigtheiten zwischen 1 und 6, und bevorzugt zwischen 1 und 3 ist; oder
- mono- oder polyhydroxylierte, und/oder mono- oder polymethoxylierte, und/oder mono- oder polyoxidierte,

und/oder mono- oder polyepoxylierte Ketten.

8. Verfahren nach einem der Ansprüche 3 bis 7, wobei der Fettstoff ein vorzugsweise natives Öl ist, ausgewählt aus Calophyllum-Öl, Himbeeröl, Kamelienöl, Nachtkerzenöl, Paranussöl, Affenbrotbaumöl und Olivenöl oder eine Mischung aus mindestens zwei dieser Öle ist; wobei das vorzugsweise native Öl, vorteilhafterweise ausgewählt ist aus Calophyllum-Öl, Himbeeröl, Kamelienöl, Nachtkerzenöl, Paranussöl und Affenbrotbaumöl oder einer Mischung aus mindestens zwei dieser Öle; wobei der Fettstoff besonders bevorzugt Calophyllum-Öl, vorzugsweise natives Calophyllum-Öl ist.

9. Verfahren nach einem der Ansprüche 3 bis 8, wobei die Diacylglycerine aus 1,2-Diacylglycerinen und 1,3-Diacylglycerinen bestehen, die jeweils durch die folgenden Formeln (I) und (II) dargestellt werden:

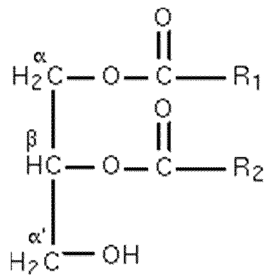

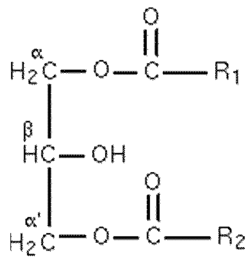

(I) (II)

worin R1 und R2 Folgendes sind:

- aliphatische Kohlenwasserstoffketten einer Fettsäure mit einer Kohlenstoffzahl zwischen 12 und 26, vorteilhafterweise zwischen 16 und 20, und besonders bevorzugt zwischen 16 und 18; wobei die aliphatische Kohlenwasserstoffkette linear oder verzweigt, vorteilhafterweise linear, gesättigt oder ungesättigt, vorteilhafterweise ungesättigt, vorzugsweise mit einer Anzahl von Ungesättigtheiten zwischen 1 und 6, und bevorzugt zwischen 1 und 3 ist; oder
- mono- oder polyhydroxylierte, und/oder mono- oder polymethoxylierte, und/oder mono- oder polyoxidierte, und/oder mono- oder polyepoxylierte Ketten.

10. Verfahren nach einem der Ansprüche 3 bis 9, wobei die Diacylglycerine vorwiegend und vorzugsweise im Wesentlichen aus 1,2-Diacylglycerinen bestehen, die durch die folgende Formel (I) dargestellt werden:

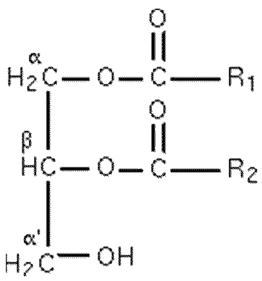

(I)

worin R1 und R2 Folgendes sind:

- aliphatische Kohlenwasserstoffketten einer Fettsäure mit einer Kohlenstoffzahl zwischen 12 und 26, vorteilhafterweise zwischen 16 und 20, und besonders bevorzugt zwischen 16 und 18; wobei die aliphatische Kohlenwasserstoffkette linear oder verzweigt, vorteilhafterweise linear, gesättigt oder ungesättigt, vorteilhafterweise ungesättigt, vorzugsweise mit einer Anzahl von Ungesättigtheiten zwischen 1 und 6, und bevorzugt zwischen 1 und 3 ist; oder
- mono- oder polyhydroxylierte, und/oder mono- oder polymethoxylierte, und/oder mono- oder polyoxidierte, und/oder mono- oder polyepoxylierte Ketten.

**11.** Verfahren zur Herstellung eines mit Fettalkoholen angereicherten Wachses, bei dem das Verfahren nach einem der Ansprüche 3 bis 5 eingesetzt wird, wobei als Fettstoff ein Wachs verwendet werden wird und anstelle eines mit Diacylglycerinen angereicherten Fettstoffs ein mit Fettalkoholen, vorzugsweise mit Fettalkoholen der Formel (III) angereichertes Wachs erhalten wird:

(III) R" - OH

worin R" für Folgendes steht:

- die aliphatische Kohlenwasserstoffkette eines Fettalkohols mit 10 bis 34 Kohlenstoffatomen, vorzugsweise 12 bis 26 Kohlenstoffatomen und besonders bevorzugt 16 bis 22 Kohlenstoffatomen;
- wobei die aliphatische Kohlenwasserstoffkette linear oder verzweigt, vorteilhafterweise linear, gesättigt oder ungesättigt, vorzugsweise mit maximal 6 Ungesättigtheiten, ist; oder
- eine mono- oder polyhydroxylierte und/oder mono- oder polyhydroxylierte und/oder mono- oder polymethoxylierte und/oder mono- oder poly-ethoxylierte und/oder mono- oder polyepoxylierte Kette.

**12.** Verfahren nach Anspruch 11, wobei das Wachs ausgewählt ist aus:

- einem Wachs tierischen Ursprungs, wie zum Beispiel Bienenwachs,
- einem Wachs mineralischen Ursprungs, oder vorzugsweise,
- einem Wachs pflanzlichen Ursprungs, wie Jojoba-, Carnauba-, Candelillawachs,
- oder Mischung(en) davon; wobei dieses Wachs vorteilhafterweise Jojobawachs ist.

**13.** Mit Diacylglycerinen angereicherter Fettstoff, erhalten durch das Verfahren nach einem der Ansprüche 3 bis 10, wobei der Fettstoff ein natives Öl ist, ausgewählt aus Calophyllum-Öl, Himbeeröl, Kamelienöl, Nachtkerzenöl, Paranussöl, Affenbrotbaumöl oder einer Mischung aus mindestens zwei dieser Öle; wobei das Öl vorteilhafterweise ausgewählt ist aus Calophyllum-Öl, Himbeeröl, Kamelienöl, Nachtkerzenöl, Paranussöl und Affenbrotbaumöl oder einer Mischung aus mindestens zwei dieser Öle; wobei der Fettstoff besonders bevorzugt Calophyllum-Öl ist.

**14.** Mit Diacylglycerinen angereicherter Fettstoff nach Anspruch 13, wobei der Fettstoff einen Diacylglyceringehalt zwischen 5 % und 30 %, vorzugsweise zwischen 7 % und 18 %, eine Säurezahl zwischen 15 und 50 mg/g und eine Peroxidzahl zwischen 5 und 30 mg/g, vorzugsweise weniger als 20 mg/g, aufweist.

**15.** Zusammensetzung, umfassend eine Mischung aus mindestens einem mit Diacylglycerinen angereicherten Fettstoff nach einem der Ansprüche 13 oder 14 und mindestens einem mit Fettalkoholen angereicherten Wachs, erhalten nach dem Verfahren nach einem der Ansprüche 11 oder 12, in einem Gewichtsverhältnis von mit Fettalkoholen angereichertem Wachs zu mit Diacylglycerinen angereichertem Fettstoff zwischen 0,01 und 0,11, vorzugsweise zwischen 0,02 und 0,08, und vorzugsweise von 0,03.

**16.** Kosmetische Verwendung einer wirksamen Menge mindestens eines mit Diacylglycerinen angereicherten Fettstoffs nach einem der Ansprüche 13 oder 14 und mindestens eines mit Fettalkoholen angereicherten Wachses, das nach dem Verfahren nach einem der Ansprüche 11 oder 12 erhalten wurde, oder einer Zusammensetzung nach Anspruch 15 durch Auftragen auf die Haut zur Verzögerung des Auftretens von oder zur Begrenzung der Anzeichen der Hautalterung, ausgewählt aus Falten und feinen Linien, Pigmentstörungen wie Altersflecken oder glanzlosem Teint, Tränensäcken, dunklen Augenringen, Verlust von Elastizität, Festigkeit und/oder Tonus der Haut, Hautverdünnung oder jeder inneren Hautschädigung infolge von Umweltbelastungen wie Umweltverschmutzung bei einem gesunden menschlichen oder tierischen Individuum, vorzugsweise einem Menschen.

**17.** Topische Zusammensetzung, umfassend eine wirksame Menge mindestens eines mit Diacylglycerinen angereicherten Fettstoffs nach einem der Ansprüche 13 oder 14 und mindestens eines mit Fettalkoholen angereicherten Wachses, erhalten nach dem Verfahren nach einem der Ansprüche 11 oder 12, oder einer Zusammensetzung nach Anspruch 15, zur Verwendung beim Schutz der Haut und/oder der Hautanhangsgebilde gegen äußere Einflüsse aufgrund von UVA- und UVB-Strahlung bei einem gesunden menschlichen oder tierischen Individuum.

## Claims

**1.** Method for obtaining a water-insoluble fraction of *Carica papaya* sap, enriched with *Carica papaya* lipase, said

method comprising the following steps:

a) suspending dried raw *Carica papaya* sap in distilled water in a weight ratio of dried raw *Carica* papaya/distilled water of between 0.01 and 0.5, preferably between 0.05 and 0.25, preferably between 0.08 and 0.2; and particularly preferably 0.1,
b) centrifuging the suspension obtained in step a) so as to obtain a pellet containing said water-insoluble fraction of the *Carica papaya* sap for a time period of between 5 minutes and 90 minutes, preferably between 15 minutes and 60 minutes, preferably between 20 and 40 minutes, and at a rotation speed of between 2000 and 6000 rpm, preferably between 3000 and 5000 rpm, preferably 4000 rpm,
c) recovering the pellet containing said water-insoluble fraction of the *Carica papaya* sap, enriched with *Carica papaya* lipase,

said method comprising, after step a) and before step b), the following step:

a') stirring the suspension obtained in step a) for a time period of between 15 minutes and 240 minutes, advantageously for a time period of between 30 minutes and 180 minutes, preferably between 60 minutes and 150 minutes, and particularly preferably 120 minutes, and at a temperature of between 10°C and 30°C, advantageously at a temperature of between 15°C and 25°C, preferably at room temperature,
said method comprising, after step b) and before step c), the following step:
b') drying the moist pellet until a particulate powder is obtained; the water-insoluble fraction of *Carica papaya* sap, enriched with *Carica papaya* lipase, being recovered, in step c), in the form of particulate powder.

**2.** Use of the water-insoluble fraction of *Carica papaya* obtained by the method according to claim 1 to enrich a fat with diacylglycerols and/or a wax with fatty alcohols.

**3.** Method for preparing a diacylglycerol-enriched fat, said method comprising the following steps:

i) mixing a fat with water or with a saline solution preferably containing a divalent ion, such as a calcium or magnesium ion, in a volume ratio of water or saline solution/fat of between 0.005 and 0.5, preferably between 0.01 and 0.4,
ii) keeping this mixture under agitation until an emulsion is obtained between the fat and the water, at a temperature of between 30°C and 70°C, advantageously at 50°C, for a time period of between 1 and 30 minutes, preferably between 5 and 20 minutes, preferably of 10 minutes,
iii) while still under agitation and at said temperature, bringing into contact the mixture obtained in the preceding step with a given volume of the water-insoluble fraction of the *Carica papaya* sap obtained by the method according to claim 1, in a volume ratio of water-insoluble fraction of the *Carica papaya* sap/fat used of between 0.01 and 0.2, advantageously between 0.05 and 0.015, preferably of 0.1, and
iv) maintaining said temperature and agitation for a time period of between one hour and six hours, advantageously between two hours and five hours, preferably for four hours, so as to obtain the diacylglycerol-enriched fat.

**4.** Method according to the preceding claim comprising, after step iv), the following step:
v) filtering the mixture obtained in step iv), preferably using at least one gradient filter or at least one superposition of at least two filters of decreasing porosity, from 500 μm to 250 μm, so as to separate said water-insoluble fraction of *Carica papaya* sap, enriched in *Carica papaya* lipase, from the reaction medium containing the diacylglycerol-enriched fat.

**5.** Method according to claim 4, said method comprising, after step v), at least one of the following two steps, and advantageously both of them:

- removing residual water present in the reaction medium containing the diacylglycerol-enriched fat by means of a drying agent, such as anhydrous magnesium sulfate, and/or
- deodorising and/or improving the shine of the diacylglycerol-enriched fat by means of an activated carbon.

**6.** Method according to one of claims 3 to 5, wherein said fat is an oil of animal, plant and/or marine origin, virgin oil or refined oil, advantageously virgin oil, said oil preferably being of plant and/or marine origin, preferably of plant origin.

**7.** Method according to one of claims 3 to 6, wherein said fat is an oil comprising triglycerides having:

- aliphatic hydrocarbon chains of a fatty acid having a carbon number of between 12 and 26, advantageously between 16 and 20, particularly preferably between 16 and 18; said aliphatic hydrocarbon chain being linear or branched, advantageously linear, saturated or unsaturated, advantageously unsaturated, preferably with a number of unsaturations of between 1 and 6, preferably between 1 and 3; or
- mono- or polyhydroxylated, and/or mono- or polymethoxylated, and/or mono- or polyoxidised, and/or mono- or polyepoxylated chains.

**8.** Method according to one of claims 3 to 7, wherein said fat is an oil, preferably virgin oil, selected from calophyllum inophyllum oil, raspberry oil, camellia oil, evening primrose oil, Brazil nut oil, baobab oil and olive oil, or a mixture of at least two of these oils; said oil, preferably virgin oil, being selected advantageously from calophyllum inophyllum oil, raspberry oil, camellia oil, evening primrose oil, Brazil nut oil and baobab oil, or a mixture of at least two of these oils; said fat particularly preferably being calophyllum inophyllum oil, preferably virgin calophyllum inophyllum oil.

**9.** Method according to one of claims 3 to 8, wherein said diacylglycerols consist of 1,2-diacylglycerols and 1,3-diacylglycerols respectively represented by the following formulas (I) and (II):

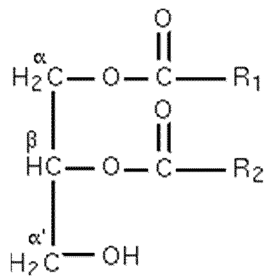

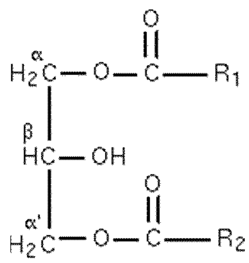

(I) (II)

in which R1 and R2 are:

- aliphatic hydrocarbon chains of a fatty acid having a carbon number of between 12 and 26, advantageously between 16 and 20, and particularly preferably between 16 and 18; said aliphatic hydrocarbon chain being linear or branched, advantageously linear, saturated or unsaturated, advantageously unsaturated, preferably with a number of unsaturations of between 1 and 6, preferably between 1 and 3; or
- mono- or polyhydroxylated, and/or mono- or polymethoxylated, and/or mono- or polyoxidised, and/or mono- or polyepoxylated chains.

**10.** Method according to one of claims 3 to 9, wherein said diacylglycerols comprise primarily, and preferably consist mainly of 1,2-diacylglycerols represented by the following formula (I):

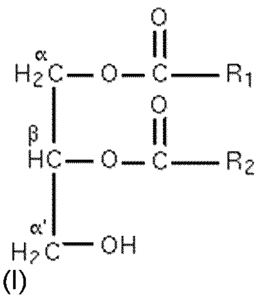

(I)

in which R1 and R2 are:

- aliphatic hydrocarbon chains of a fatty acid having a carbon number of between 12 and 26, advantageously between 16 and 20, and particularly preferably between 16 and 18; said aliphatic hydrocarbon chain being linear or branched, advantageously linear, saturated or unsaturated, advantageously unsaturated, preferably with a number of unsaturations of between 1 and 6, preferably between 1 and 3; or
- mono- or polyhydroxylated, and/or mono- or polymethoxylated, and/or mono- or polyoxidised, and/or mono-

or polyepoxylated chains.

**11.** Method for preparing a fatty alcohol-enriched wax, implementing the method according to one of claims 3 to 5, wherein a wax is used as a fat, and, in place of a diacylglycerol-enriched fat, a fatty alcohol-enriched wax is obtained, the fatty alcohols advantageously being of formula (III):

(III) R" - OH

in which R" represents:

- an aliphatic hydrocarbon chain of a fatty alcohol containing from 10 to 34 carbon atoms, preferably from 12 to 26 carbon atoms, and particularly preferably from 16 to 22 carbon atoms;
- said aliphatic hydrocarbon chain being linear or branched, advantageously linear, saturated or unsaturated, preferably with a maximum of 6 unsaturations; or
- a mono- or polyhydroxylated, and/or mono- or polymethoxylated, and/or mono-or polyoxidised, and/or mono- or polyepoxylated chain.

**12.** Method according to claim 11, wherein said wax is selected from:

- a wax of animal origin, for example beeswax,
- a wax of mineral origin, or, preferably,
- a wax of plant origin, such as jojoba wax, carnauba wax, candelilla wax,
- or mixture(s) thereof; said wax being advantageously jojoba wax.

**13.** Diacylglycerol-enriched fat, obtained by the method according to one of claims 3 to 10, wherein said fat is a virgin oil selected from calophyllum inophyllum oil, raspberry oil, camellia oil, evening primrose oil, Brazil nut oil, baobab oil or a mixture of at least two of these oils; said oil being selected advantageously from calophyllum inophyllum oil, raspberry oil, camellia oil, evening primrose oil, Brazil nut oil and baobab oil, or a mixture of at least two of these oils; said fat particularly preferably being calophyllum inophyllum oil.

**14.** Diacylglycerol-enriched fat according to claim 13, said fat having a diacylglycerol content of between 5% and 30%, preferably between 7% and 18%, an acidity index of between 15 and 50 mg/g, and a peroxide index of between 5 and 30 mg/g, preferably lower than 20 mg/g.

**15.** Composition comprising a blend of at least one diacylglycerol-enriched fat according to one of claims 13 or 14 and at least one fatty alcohol-enriched wax obtained by the method according to one of claims 11 or 12, in a ratio by weight of fatty alcohol-enriched wax/diacylglycerol-enriched fat of between 0.01 and 0.11, preferably between 0.02 and 0.08, preferably of 0.03.

**16.** Cosmetic use of an effective amount of at least one diacylglycerol-enriched fat according to one of claims 13 or 14 and of at least one fatty alcohol-enriched wax obtained by the method according to one of claims 11 or 12, or of a composition according to claim 15, by dermal application, to delay the appearance or limit the signs of skin aging, selected from fine lines and wrinkles, pigmentary defects such as age spots or lack of radiance, bags under the eyes, dark circles, withering, loss of elasticity, firmness and/or skin tone, thinning of the skin or any internal damage to the skin resulting from environmental stresses such as pollution and UV radiation, in a healthy human or animal individual, preferably human.

**17.** Topical composition comprising an effective amount of at least one diacylglycerol-enriched fat according to one of claims 13 or 14 and of at least one fatty alcohol-enriched wax obtained by the method according to one of claims 11 or 12, or of a composition according to claim 15, for use to protect the skin and/or appendages against external aggressions caused by UVA and UVB rays, in a healthy human or animal individual.

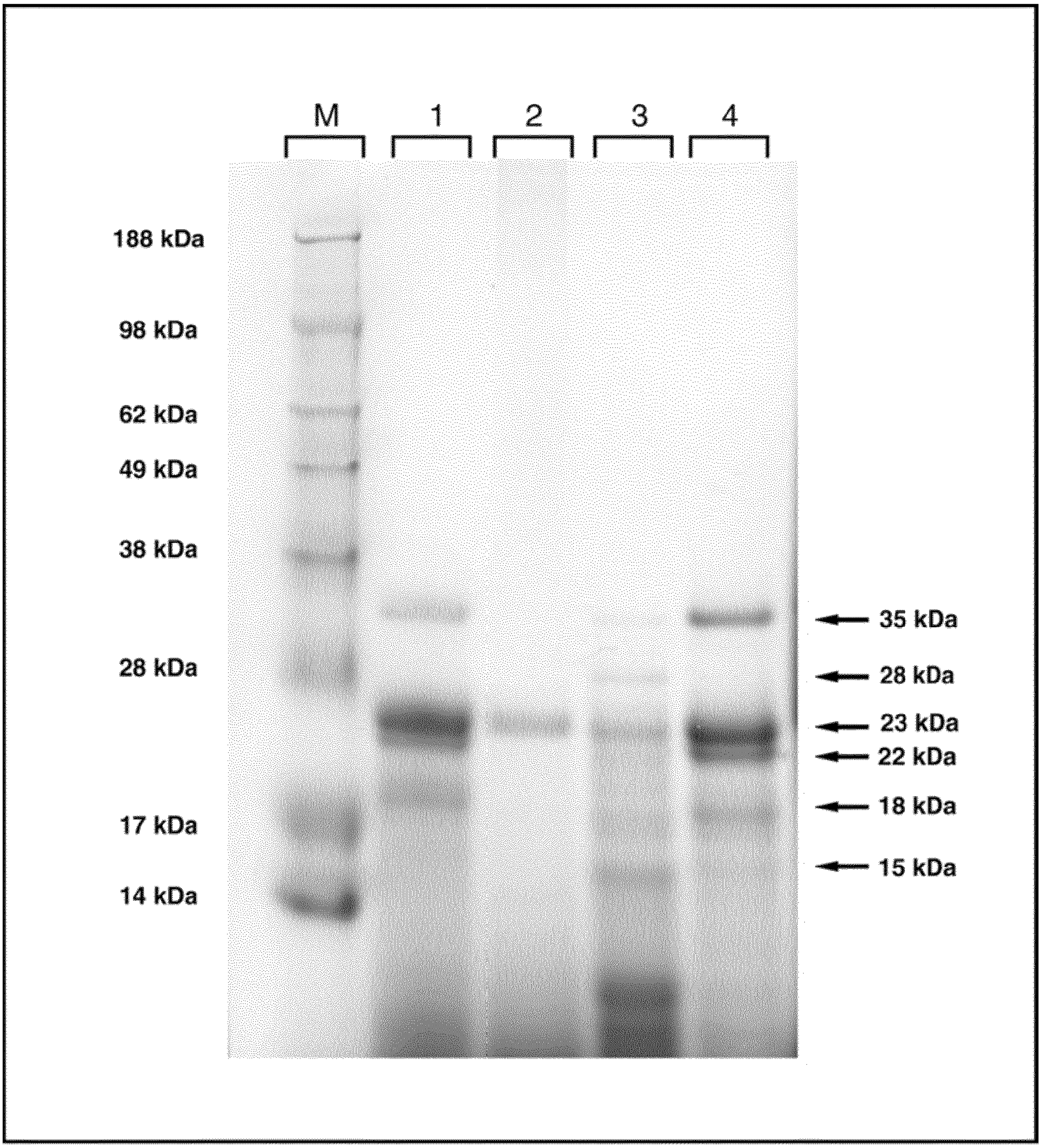
M
1
2
3
4
188 kDa
98 kDa
62 kDa
49 kDa
38 kDa
28 kDa
17 kDa
14 kDa
35 kDa
28 kDa
23 kDa
22 kDa
18 kDa
15 kDa

FIG. 1

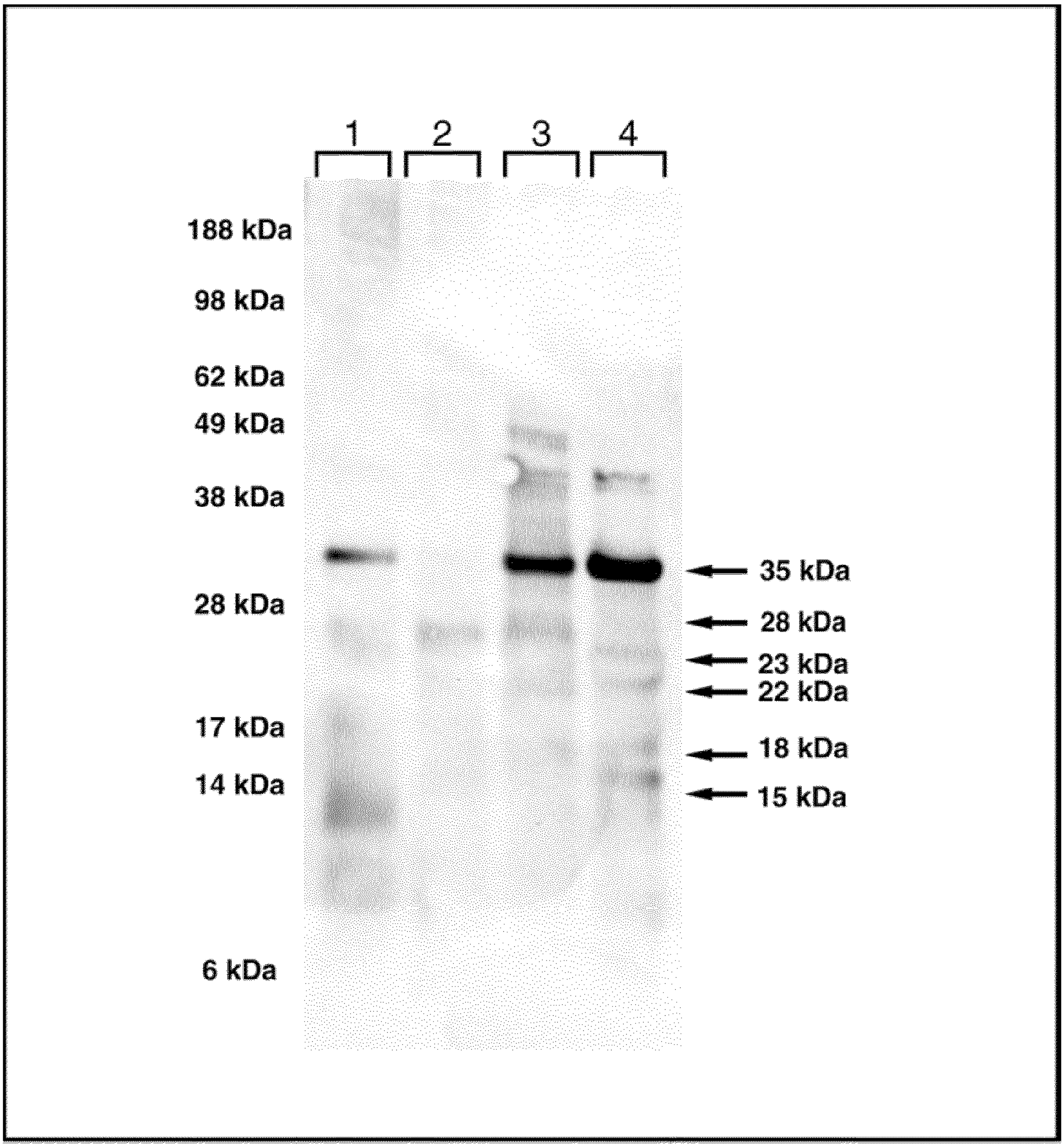
1
2
3
4
188 kDa
98 kDa
62 kDa
49 kDa
38 kDa
28 kDa
17 kDa
14 kDa
6 kDa
35 kDa
28 kDa
23 kDa
22 kDa
18 kDa
15 kDa

FIG. 2

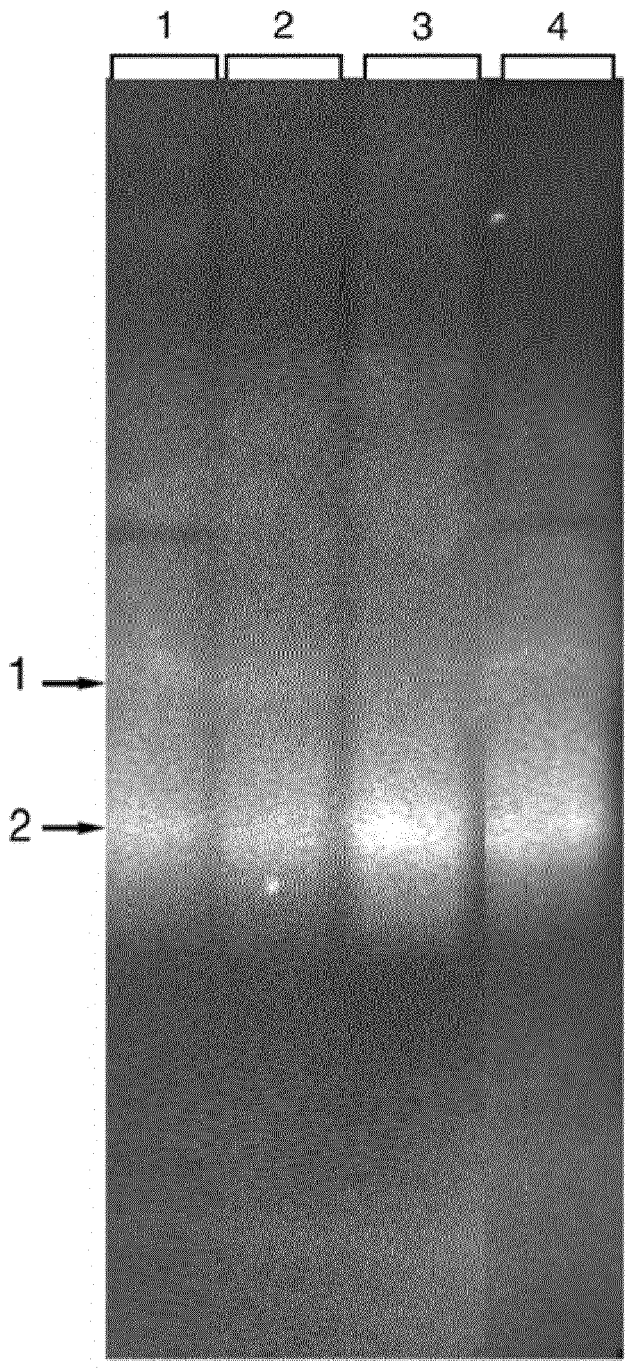


FIG. 3

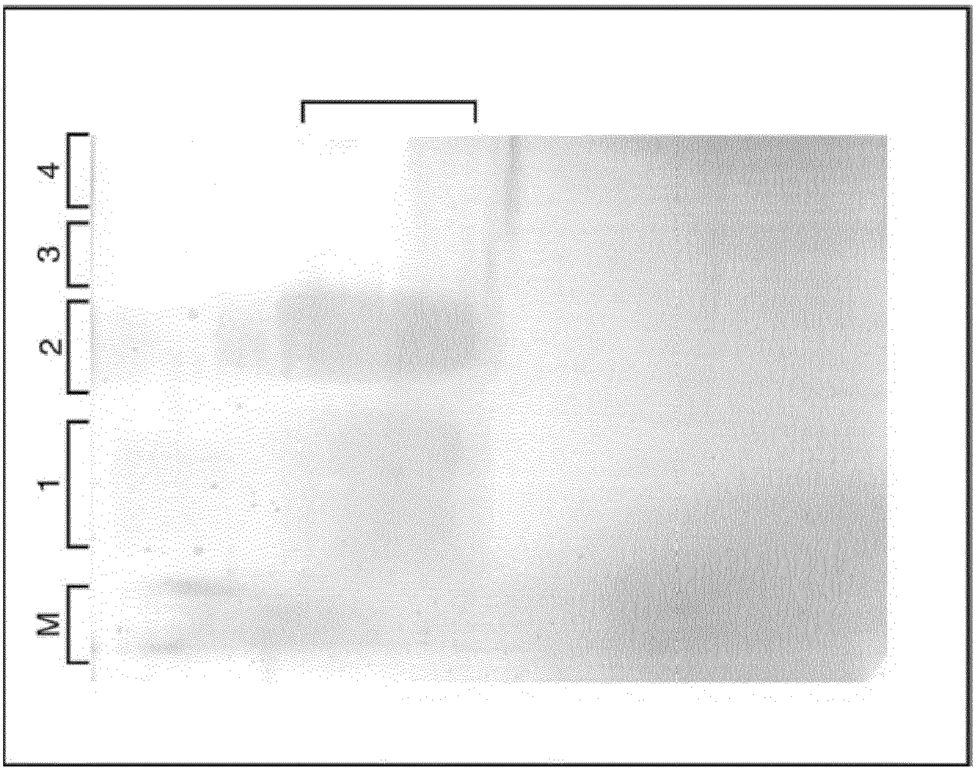


FIG. 4B

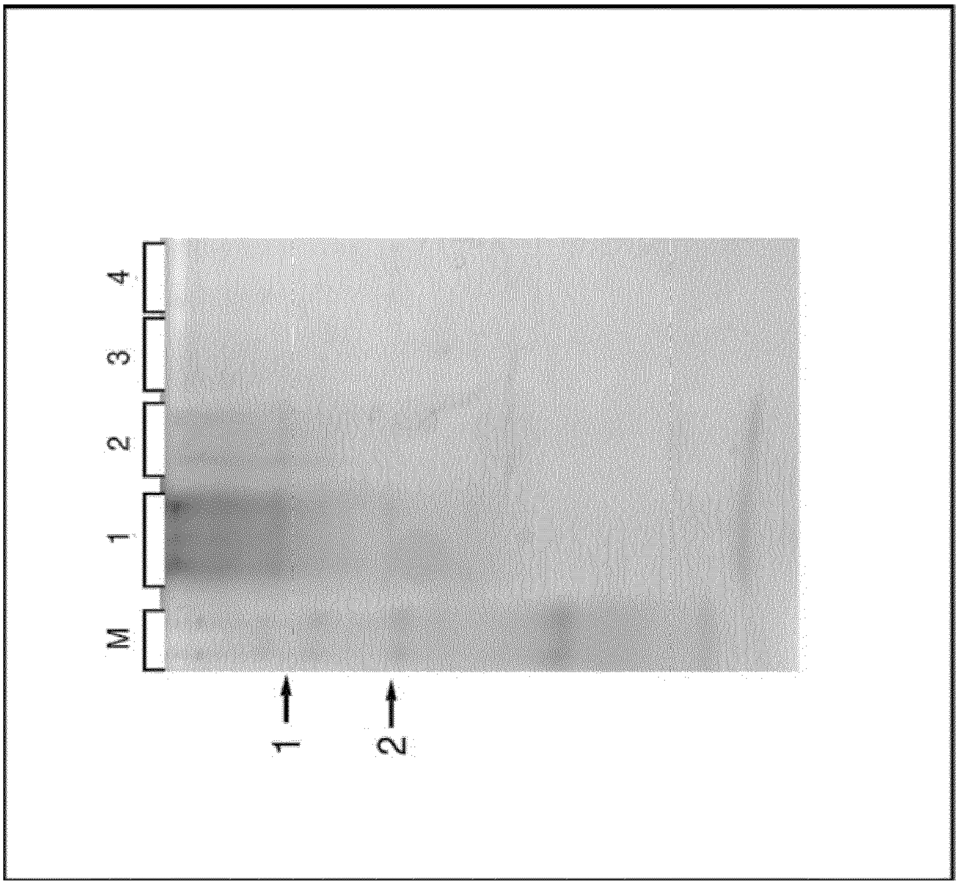


FIG. 4A

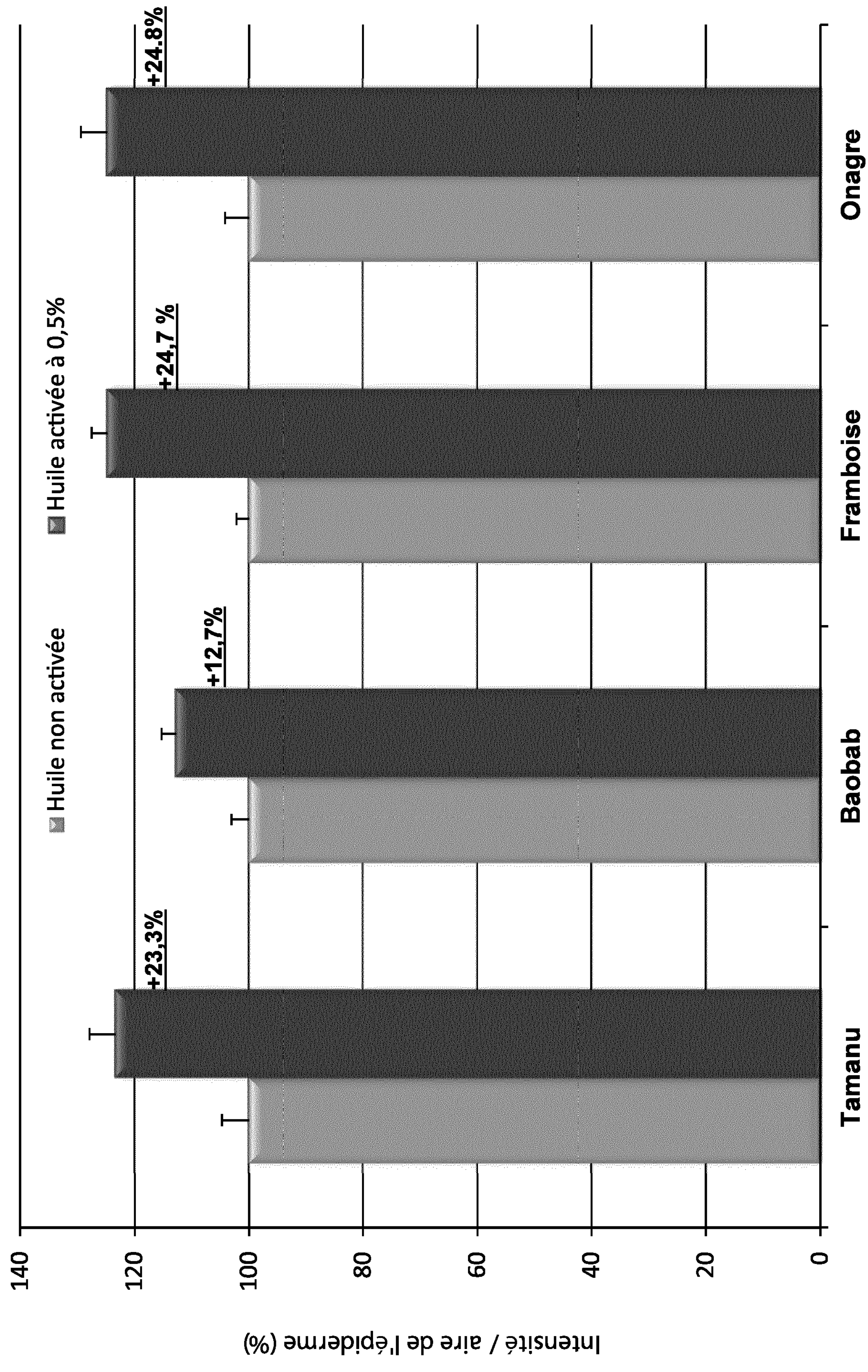
Huile non activée
Huile activée à 0,5%
Intensité / aire de l'épiderme (%)
140
120
100
80
60
40
20
0
Tamanu
Baobab
Framboise
Onagre
+23,3%
+12,7%
+24,7 %
+24.8%

FIG. 5

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- FR 2004000944 W
- WO 2004093832 A2
- WO 2004093832 A
- FR 2827170
- FR 2837098
- FR 2841781
- FR 2846883
- FR 2817748
- FR 2956818
- FR 2951946
- FR 2944526

### Littérature non-brevet citée dans la description


- **VILLENEUVE et al.** *JAOCS,* 1995, vol. 72 (6), 753
- **FUERTES L. ; SANTONJA C. ; KUTZNER H. ; REQUENA L.** Immunohistochemistry in Dermatopathology : a review of the most commonly used antibodies (Part I). *Actas Dermo-Sifiliográficas.,* 2013, vol. 104 (2), 99-127
- **PALADINI R.D. ; COULOMBE P.A.** The functional diversity of epidermal keratins revealed by the partial rescue of the keratin 14 null phenotype by keratin 16. *The Journal of Cell Biology,* 1999, vol. 146 (5), 1185-1201